# EUROPÄISCHE PATENTANMELDUNG

(11) **EP 4 250 177 A1**
(43) Veröffentlichungstag der Anmeldung: **27.09.2023**
(21) Anmeldenummer: 23162342.2
(22) Anmeldetag: 16.03.2023
(51) Int. Cl.: G06K 19/00, G16H 10/65, G06K 19/077

(54) **ELEKTRONISCHE GESUNDHEITSKARTE**

(30) Priorität: 23.03.2022 DE 102022106878
(71) Anmelder: Bundesdruckerei GmbH, 10969 Berlin (DE)
(72) Erfinder: RÜCKRIEMEN, Jörg, verstorben (DE)
(74) Vertreter: Richardt Patentanwälte PartG mbB

(57) **Zusammenfassung**

Die Erfindung betrifft eine elektronische Gesundheitskarte (100) mit einem Kartenkörper (101), welcher in einem Ausgabezustand einen markierten Abschnitt (120) umfasst. Ein Vorhandensein des markierten Abschnitts (120) zeigt eine Zustimmung des Inhabers zu einer Organspende an, wohingegen ein Fehlen des markierten Abschnitts (120) durch ein Entfernen eine Ablehnung des Inhabers zu der Organspende anzeigt.

## Beschreibung

Die Erfindung betrifft eine elektronische Gesundheitskarte, ein Terminal zum Auslesen einer elektronischen Gesundheitskarte sowie ein System aus einer elektronischen Gesundheitskarte und einem Terminal.

Zur Erklärung einer Zustimmung oder Ablehnung einer Bereitschaft zu einer Organ- oder Gewebespende wird zumeist ein sogenannter Organspendeausweis verwendet. Der in Deutschland aktuell meistgenutzte Organspendeausweis wird von der Bundeszentrale für gesundheitliche Aufklärung herausgegeben und hat das Scheckkartenformat. Auf der Vorderseite des Organspendeausweises sind Name und Anschrift des Ausweisinhabers einzutragen.

Auf der Rückseite kann der Ausweisinhaber seine persönliche Erklärung zur Organspende ab, indem er aus den dort genannten Optionen die für ihn gültige wählt. Die Angaben können bei Bedarf in einem zusätzlichen Absatz Anmerkungen/Besondere Hinweise ergänzt werden und gelten durch die Unterschrift des Ausweisinhabers und das gesetzte Datum der Ausstellung als bestätigt. Es gibt den Organspendeausweis sowohl aus Papier, als auch in Form einer Plastikkarte. Mit dieser Willenserklärung erklärt die ausstellende Person, ob sie im Todesfall mit einer Überlassung sämtlicher oder einiger Organe und Gewebe zu Spendezwecken einverstanden ist oder eine entsprechende Organentnahme ablehnt. Bei einem solchen Organspendeausweis handelt es sich aber um kein amtliches Formular. Ebenso ist es möglich die Zustimmung oder Ablehnung zu einer Organspende formlos etwa auf einem Blatt Papier festzuhalten.

Damit ein so festgelegter Wunsch bezüglich einer Zustimmung oder Ablehnung einer Organspende im Falle eines unvorhergesehenen Todes berücksichtigt werden kann, ist es im Allgemeinen notwendig, den entsprechenden Zettel nach Möglichkeit stets bei sich zu tragen. Dies ist im Alltag jedoch oftmals nicht der Fall. Es gibt zwar Pläne zum Aufbau eines zentralen digitalen Organspenderregisters. Ein solches Register erfordert aber zum effektiven Schutz der darin gespeicherten Daten und zur eindeutigen Identifikation der Personen, welche dort Ihre Daten und ihren Wunsch bezüglich einer Organspende ablegen, aufwendige kryptographische Sicherungsmechanismen. Die daraus resultierenden Hürden können dazu führen, dass die Bereitschaft zu einer Nutzung des entsprechenden Registers, insbesondre zur Erklärung einer Zustimmung zu einer Organsende mittels des Registers, sinken könnte.

Der Erfindung liegt daher die Aufgabe zugrunde, eine effektive, effiziente und leicht zu nutzende Möglichkeit zum Erklären einer Zustimmung oder einer Ablehnung zu einer Organspende zu schaffen.

Die der Erfindung zugrundeliegende Aufgabe wird jeweils mit den Merkmalen der unabhängigen Patentansprüche gelöst. Ausführungsformen der Erfindung sind in den abhängigen Patentansprüchen angegeben.

Ausführungsformen umfassen eine elektronische Gesundheitskarte. Die Gesundheitskarte umfasst einen Kartenkörper mit einem Prozessor, einem Speicher mit Programminstruktionen und einer Kommunikationsschnittstelle. In dem Speicher sind ferner personenbezogene und krankenversicherungsbezogene Daten eines Inhabers der elektronischen Gesundheitskarte gespeichert. Der Prozessor ist dazu konfiguriert, die gespeicherten Daten unter Verwendung der Programminstruktionen zum Auslesen über die Kommunikationsschnittstelle bereitzustellen.

Der Kartenkörper umfasst ferner in einem Ausgabezustand einen markierten Abschnitt. Das Vorhandensein des markierten Abschnitts zeigt eine Zustimmung des Inhabers zu einer Organspende an. Ein Entfernen des markierten Abschnitts zeigt eine Ablehnung des Inhabers zu der Organspende an.

Ausführungsformen können den Vorteil haben, dass für den Inhaber einer elektronischen Gesundheitskarte eine einfache Entscheidungsmöglichkeit für oder gegen eine Organspende bereitstellen. Eine elektronische Gesundheitskarte besitzt jeder Krankenversicherte und diese wird im Falle eines Todes des Inhabers sowieso benötigt. Ferner wird die die elektronische Gesundheitskarte in der Regel ständig mitgeführt.

Nach Ausführungsformen wird ein Organspenderausweis somit in eine elektronische Gesundheitskarte integriert. Dabei besteht der Vorteil in einer Implementierung der Willenserklärung in Form eines nicht abgetrennten oder abgetrennten Abschnitts des Kartenkörpers der elektronischen Gesundheitskarte darin, dass dies von dem Inhaber der Gesundheitskarte selbst jederzeit vorgenommen werden kann. Der Inhaber benötigt dazu keinen Zugriff auf die in dem Speicher der elektronischen Gesundheitskarte gespeicherten und vor Zugriffen kryptographisch geschützten Daten. Zudem ist das Abgeben der entsprechenden Willenserklärung technisch einfach umzusetzen, ohne dass zusätzliche technische Hilfsmittel notwendig wären. Allenfalls beispielsweise eine Schere kann zum Abtrennen des markierten Abschnitts des Kartenkörpers zum Einsatz kommen. Durch ein solches Abtrennen des markierten Abschnitts wird die elektronische Gesundheitskarte aber insbesondere nicht ungültig, sondern es werden vielmehr sozusagen physisch zusätzliche Informationen den Kartenkörper der elektronischen Gesundheitskarte kodiert, nämlich eine Zustimmung zu einer Organspende. Somit ist weder ein separater Organspenderausweis, noch ein zentrales Organspenderregister notwendig. Eine durch Abtrennung des markierten Abschnitts erklärte Ablehnung einer Organspende kann durch Unbefugte nicht rückgängig gemacht werden. Soll eine solche erklärte Ablehnung wieder rückgängig gemacht werden ist eine neue elektronische Gesundheitskarte auszustellen, welche die bisherige Gesundheitskarte ersetzt.

Beispielsweise kann es sich bei dem markierten Abschnitt um eine Ecke des Kartenkörpers handeln. Beispielsweise kann der markierte Abschnitt farblich hervorgehoben sein, sodass dessen Vorhandensein oder Fehlen leichter ersichtlich wird. Beispielsweise kann der markierte Abschnitt grün markiert sein, um seine positive Bedeutung hinsichtlich einer Organspende intuitiv zu vermitteln. Ferner kann der markierte Abschnitt beispielsweise mit einer auf Beschriftung, wie etwa "Organspende ja" beschriftet sein.

Beispielsweise kann der markierte Abschnitt dazu vorgesehen sein, zu einer Ablehnung einer Organspende mit einem Hilfsmittel, wie etwa einer Schere, abgetrennt zu werden. markiert.

Unter einer "Kommunikationsschnittstelle" wird hier beispielsweise eine Schnittstelle verstanden, über die Daten empfangen und gesendet werden können, wobei die Kommunikationsschnittstelle kontaktbasiert oder kontaktlos konfiguriert sein kann.

Eine Kommunikation kann beispielsweise über ein Netzwerk erfolgen. Unter einem "Netzwerk" wird hier jedes Übertragungsmedium mit einer Anbindung zur Kommunikation verstanden, insbesondere eine lokale Verbindung oder ein lokales Netzwerk, insbesondere ein Local Area Network (LAN), ein privates Netzwerk, insbesondere ein Intranet, und ein digitales privates Netzwerk (Virtual Private Network - VPN). Beispielsweise kann ein Computersystem eine Standardfunkschnittstelle zur Anbindung an ein WLAN aufweisen. Ferner kann es sich um ein öffentliches Netzwerk, wie beispielsweise das Internet handeln.

Eine kontaktlose Kommunikation mit der elektronischen Gesundheitskarte ist beispielsweise mittels Near Field Communication (NFC) möglich. Hierbei handelt es sich um eine auf der RFID-Technologie basierende Kommunikation zum kontaktlosen Austausch von Daten per elektromagnetischer Induktion mittels loser gekoppelter Spulen über kurze Strecken, von beispielsweise wenigen Zentimetern. NFC kann beispielsweise gemäß einer der Normen ISO 14443, 18092, 21481, ECMA 340, 352, 356, 362 bzw. ETSI TS 102 190 implementiert sein.

Die Kommunikationsschnittstelle der elektronischen Gesundheitskarte umfasst beispielsweise eine Antenne zur kontaktlosen Kommunikation. Die Antenne umfasst beispielsweise eine Induktionsspule. Die Induktionsspule kann ferner konfiguriert sein zur externen Energieversorgung der elektronischen Gesundheitskarte, beispielsweise mittels Energy Harvesting. Beispielsweise ist die Induktionsspule dazu konfiguriert, dass ein Terminal Energie in die elektronische Gesundheitskarte einkoppelt.

Unter einem "Prozessor" wird hier und im Folgenden eine Logikschaltung verstanden, die zur Ausführung von Programminstruktionen dient. Die Logikschaltung kann auf einem oder mehreren diskreten Bauelementen implementiert sein, insbesondere auf einem Chip. Ein Prozessor umfasst beispielsweise ein Rechenwerk, ein Steuerwerk, Register und Datenleitungen zur Kommunikation mit anderen Komponenten. Insbesondere wird unter einem "Prozessor" ein Mikroprozessor oder ein Mikroprozessorsystem aus mehreren Prozessorkernen und/oder mehreren Mikroprozessoren verstanden.

Unter einem "Speicher" wird hier insbesondere ein nichtflüchtiger Speicher verstanden. Unter einem "nichtflüchtigen Speicher" wird hier beispielsweise ein elektronischer Speicher zur dauerhaften Speicherung von Daten verstanden. Ein nichtflüchtiger Speicher kann als nichtänderbarer Speicher konfiguriert sein, der auch als Read-Only Memory (ROM) bezeichnet wird, oder als änderbarer Speicher, der auch als Non-Volatile Memory (NVM) bezeichnet wird. Insbesondere kann es sich hierbei um ein EEPROM, beispielsweise ein Flash-EEPROM, kurz als Flash bezeichnet, handeln. Ein nichtflüchtiger Speicher zeichnet sich dadurch aus, dass die darauf gespeicherten Daten auch nach Abschalten der Energieversorgung erhalten bleiben.

Unter einem "geschützten Speicherbereich" wird hier ein Bereich eines elektronischen Speichers verstanden, auf den ein Zugriff, das heißt ein Lesezugriff oder ein Schreibzugriff, nur über einen Prozessor der elektronischen Gesundheitskarte möglich ist. Nach Ausführungsformen ist der Zugriff von dem bzw. über den mit dem Speicher gekoppelten Prozessor nur dann möglich, wenn eine hierzu erforderliche Bedingung erfüllt ist. Hierbei kann es sich zum Beispiel um eine kryptographische Bedingung, insbesondere eine erfolgreiche Authentisierung und/oder eine erfolgreiche Berechtigungsprüfung, handeln. Eine solche Prüfung kann beispielsweise auf einer elektronischen Signatur mit einem Signaturschlüssel beruhen.

Asymmetrische Schlüsselpaare werden für eine Vielzahl von Kryptosystemen eingesetzt und spielen auch bei der Signatur elektronischer Dokumente eine wichtige Rolle. Ein asymmetrisches Schlüsselpaar besteht aus einem öffentlichen Schlüssel, welcher zur Ver- und/oder Entschlüsselung von Daten verwendet wird und an Dritte weitergegeben werden darf, sowie einem privaten Schlüssel, welcher zur Ver- und/oder Entschlüsselung von Daten verwendet wird und im Regelfall geheim gehalten werden muss. Der öffentliche Schlüssel ermöglicht es jedermann, Daten für den Inhaber des privaten Schlüssels zu verschlüsseln und digitale mit dem privaten Schlüssel erstellte Signaturen zu prüfen. Ein privater Schlüssel ermöglicht es seinem Inhaber, mit dem öffentlichen Schlüssel verschlüsselte Daten zu entschlüsseln oder digitale Signaturen zu erstellen. Eine mit einem privaten Schlüssel erstellte Signatur kann mit dem zugehörigen öffentlichen Schlüssel verifiziert werden.

Die Erstellung einer digitalen Signatur, im Folgenden auch lediglich als "Signatur" bezeichnet, ist ein kryptographisches Verfahren, bei dem zu beliebigen Daten ein weiterer Datenwert, welcher als "Signatur" bezeichnet wird, berechnet wird. Bei einer Signatur kann es sich zum Beispiel um eine mit einem privaten kryptographischen Schlüssel verschlüsselten Hashwert der Ausgangsdaten handeln.

Eine symmetrische Verschlüsselung basiert auf einem symmetrischen kryptographischen Schlüssel. In diesem Fall werden Daten mit demselben symmetrischen kryptographischen Schlüssel verschlüsselt und wieder entschlüsselt.

Die personenbezogenen und versicherungsbezogenen Daten umfassen beispielsweise einen Namen einer ausstellenden Krankenkasse und ein Kennzeichen für die Kassenärztliche Vereinigung, in deren Bezirk der Versicherte seinen Wohnsitz hat, einen Familienname und einen Vorname des Versicherten, ein Geburtsdatum, das Geschlecht, eine Anschrift, eine Krankenversichertennummer, einen Versichertenstatus, einen Tag des Beginns des Versicherungsschutzes, und/oder bei einer befristeten Gültigkeit der Gesundheitskarte ein Datum des Fristablaufs.

Der Kartenkörper des elektronischen Gesundheitskarte kann aus einem Polymer gebildet sein, das ausgewählt ist aus einer Gruppe, umfassend Polycarbonat (PC), insbesondere Bisphenol A-Polycarbonat, Polyethylenterephthalat (PET), deren Derivate, wie Glykolmodifiziertes PET (PETG), Polyethylennaphthalat (PEN), Polyvinylchlorid (PVC), Polyvinylbutyral (PVB), Polymethylmethacrylat (PMMA), Polyimid (PI), Polyvinylalkohol (PVA), Polystyrol (PS), Polyvinylphenol (PVP), Polypropylen (PP), Polyethylen (PE), thermoplastische Elastomere (TPE), insbesondere thermoplastisches Polyurethan (TPU), Acrylnitril-Butadien-Styrol-Copolymer (ABS) sowie deren Derivate, und/oder Papier und/oder Pappe. Außerdem kann der Kartenkörper auch aus mehreren dieser Materialien hergestellt sein. Bevorzugt besteht es aus PC, PET und/oder PVC. Die Polymere können entweder gefüllt oder ungefüllt vorliegen. Im letzteren Falle sind sie vorzugsweise transparent oder transluzent. Falls die Polymere gefüllt sind, sind sie opak. Die vorstehenden Angaben beziehen sich sowohl auf miteinander zu verbindende Folien als auch auf Flüssigformulierungen, die auf ein Vorprodukt aufgebracht werden, wie einen Schutz- oder Decklack. Bevorzugt wird das Dokument aus einer Mehrzahl von Materialschichten bzw. Folien hergestellt, vorzugsweise mit einem Laminierverfahren, bei dem die Folien unter Druck- und Wärmeeinwirkung miteinander verschmolzen werden. Die einzelnen Folien können aus dem gleichen Material oder aus unterschiedlichen Materialien bestehen. Ferner kann eine Herstellung des Dokuments ein Bilden von Overlaylagen umfassen. Derart gebildete Overlaylagen schützen ein darunter angeordnetes Merkmal und/oder verleihen dem Dokument die erforderliche Abriebfestigkeit.

Nach Ausführungsformen ist zumindest ein Teil einer den markierten Abschnitt begrenzenden Grenzlinie als Sollbruchlinie ausgestaltet. Ausführungsformen können den Vorteil haben, dass durch eine Sollbruchlinie das Abtrenne des markierten Abschnitts erleichtert werden kann. Insbesondere kann die Sollbruchlinie derart präpariert sein, dass ein Abtrennen des markierten Abschnitts entlang der Sollbruchlinie auch ohne zusätzliche Hilfsmittel, wie etwa eine Schere leicht per Hand möglich ist.

Beispielsweise ist der markierte Abschnitt durch die Sollbruchlinie, etwa in Form einer Kerbe oder Perforation markiert, Beispielsweise ist der markierte Abschnitt zusätzlich oder alternativ farblich markiert. Beispielsweise ist eine Linie farblich markiert, entlang derer der markierte Abschnitt abzutrennen ist. Die entsprechende Linie kann beispielsweise als Sollbruchlinie ausgestaltet sein.

Eine Sollbruchlinie ist eine Linie, welche in deren Bereich der Kartenkörper durch seine Struktur, Gestalt und/oder Konstruktion, insbesondere durch eine Materialverjüngung, dazu bestimmt ist bei Belastung vorhersagbar entlang der entsprechenden Linie zu brechen. Eine solche Sollbruchlinie kann beispielsweise in Form einer Kerbe, eine Perforation oder einer Ritzspur implementiert werden. Durch eine daraus resultierende Kerbwirkung kann der Kartenkörper entlang der Sollbruchlinie entscheidend geschwächt werden.

Nach Ausführungsformen weist der Kartenkörper entlang der Sollbruchlinie eine im Verhältnis zu einem angrenzenden Bereich des Kartenkörpers reduzierte Materialstärke auf. Ausführungsformen können den Vorteil haben, dass aufgrund der Materialreduktion der Kartenkörper entlang der Sollbruchlinie geschwächt ist, wodurch ein Abtrennen des markierten Abschnitts erleichtert werden kann.

Nach Ausführungsformen ist die Sollbruchlinie in Form einer Kerbe oder Ritzlinie entlang zumindest eines Teils der Grenzlinie ausgestaltet. Nach Ausführungsformen ist die Kerbe beispielsweise mittels eines der folgenden Bearbeitungsverfahren in den Kartenkörper eingebracht: Fräsen, Laserabtragen, Einkerben, Einprägen oder Ultraschallprägen bzw.- abtragen.

Nach Ausführungsformen weist der Kartenkörper entlang der Sollbruchlinie eine Perforation auf. Ausführungsformen können den Vorteil haben, dass eine Perforation eine einfache Abtrennbarkeit des markierten Abschnitts vom Kartenkörper ermöglichen kann. Nach Ausführungsformen ist die Peroration beispielsweise mittels eines der folgenden Bearbeitungsverfahren in den Kartenkörper eingebracht: Fräsen, Laserabtragen, Einkerben, Einprägen oder Ultraschallprägen bzw.- abtragen.

Nach Ausführungsformen ist die Sollbruchlinie zu einem manuellen Abrechen des markierten Abschnitts entlang der Sollbruchlinie mittels eines Sprödbruchs konfiguriert. Ein Sprödbruch ist ein Bruch, welcher ohne oder mit geringer plastischer Verformung bei Überschreiten einer vordefinierten Spaltbruchspannung auftritt. Ausführungsformen können den Vorteil haben, dass der markierte Abschnitt ohne viel Aufwand durch Aufbringen einer Kraft von Hand, welche die vordefinierten Spaltbruchspannung überschreitet, abgebrochen werden kann.

Nach Ausführungsformen ist die Sollbruchlinie zu einem manuellen Abrechen des markierten Abschnitts entlang der Sollbruchlinie mittels eines Verformungsbruchs konfiguriert. Ein Verformungsbruch ist ein Bruch, welcher erst nach einer bzw. im Zuge einer plastischen Verformung auftritt. Ausführungsformen können den Vorteil haben, dass ein versehentliches Abrechen des markierten Abschnitts vermieden werden kann. Es ist erst eine erhebliche Verformung, beispielsweise durch ein Hin- und Herknicken des markierten Abschnitts um die Sollbruchlinie notwendig, bis das Kartenmaterial entlang der Sollbruchlinie nachgibt.

Nach Ausführungsformen handelt es sich bei den Kartenkörper um einen mehrschichtigen Verbundkörper, welcher eine Mehrzahl von Materialschichten umfasst. Nach Ausführungsformen erstreckt sich der markierte Abschnitt durch ein oder mehrerer aufeinander angeordnete Materialschichten der Mehrzahl von Materialschichten hindurch, während sich der markierte Abschnitt durch ein oder mehrere weitere Materialschichten der Mehrzahl von Materialschichten nicht hindurcherstreckt.

Ausführungsformen können den Vorteil haben, dass bei einem Entfernen des markierten Abschnitts jeweils die Teile der ein oder mehrerer aufeinander angeordnete Materialschichten entfernt werden, durch welche sich der markierte Abschnitt hindurcherstreckt. Demgegenüber bleiben diejenigen Materialschichten, durch welche sich der markierte Abschnitt nicht hindurcherstreckt, von dem Entfernen des markierten Abschnitts unberührt, d.h., intakt. Die Form der Gesundheitskarte, d.h., der geometrische Umriss bleibt somit beispielsweise unverändert. Beispielsweise entstehen durch das Entfernen des markierten Abschnitts ein Sackloch, eine Vertiefung, und/oder eine Stufe in dem Kartenkörper. Beispielsweise kann sich aus dem Fehlen des markierten Abschnitts an einem Rand des Verbundkörpers eine Stufe ergeben, ohne dass sich der geometrische Umriss der Gesundheitskarte infolge des Fehlens des markierten Abschnitts ändert. Beispielsweise kann sich aus dem Fehlen des markierten Abschnitts, welcher beabstandet von dem Rand des Verbundkörpers angeordnet ist, eine Vertiefung und/oder ein Sackloch innerhalb des Umrisses des Verbundkörpers ergeben. Beispielsweise sind die ein oder mehrerer aufeinander angeordnete Materialschichten, durch welche sich der markierte Abschnitt hindurcherstreckt, dünner, insbesondere deutlich dünner, als diejenigen Materialschichten, durch welche sich der markierte Abschnitt nicht hindurcherstreckt.

Beispielsweise umfasst der Verbundkörper wenigstens eine erste und eine zweiten Materialschicht bzw. Materiallage. Der markierte Abschnitt ist beispielsweise dazu konfiguriert im Zuge eines Entfernens aus der ersten Materialschicht herausgebrochen und/oder abgehoben zu werden. Nach dem Entfernen des markierten Abschnitts bleibt der Kartenkörper dann beispielsweise zum größten Teil erhalten, sodass etwa formfaktor-basierte Anwendungen der Gesundheitskarte problemlos weiterhin funktionieren. Beispielsweise kann vorgesehen sein, dass die erste Materialschicht dünner, insbesondere deutlich dünner, als die zweite Materialschicht ist.

Nach Ausführungsformen erstreckt sich der markierte Abschnitt durch alle Materialschichten der Mehrzahl von Materialschichten hindurch. Ausführungsform können den Vorteil haben, dass infolge des Entfernens des markierten Abschnitts ein vollständiges Stück des Kartenkörpers fehlt, d.h., ein Stück, welches Teile aller Materialschichten des Kartenkörpers umfasst. Hierdurch kann sich beispielsweise der geometrische Umriss des Kartenkörpers ändern. Beispielsweise kann sich aus dem Fehlen des markierten Abschnitts ein Durchgangsloch in dem Kartenkörper ergeben.

Nach Ausführungsformen ist der markierte Abschnitt von der Sollbruchlinie und einem Rand des Kartenkörpers begrenzt. Ausführungsformen können den Vorteil haben, dass der markierte Abschnitt, wenn er am Rand des Kartenkörpers angeordnet ist, zum Abtrenne leicht erreichbar ist. Zudem muss der markierte Abschnitt in diesem Fall nicht vollumfänglich von dem Kartenkörper getrennt werden, wodurch das Abtrennen ebenfalls erleichtert wird.

Nach Ausführungsformen wird durch ein Entfernen des markierten Abschnitts ein geometrischer Umriss des Kartenkörpers verändert. Ausführungsformen können den Vorteil haben, dass die Umrissänderung des Kartenkörpers und somit das Abtrennen des markierten Bereichs leicht erkennbar ist. Insbesondere kann das Abtrennen in diesem Fall leicht maschinelle erkannt werden, etwa bei einem Anordnen der elektronischen Gesundheitskarte in einer Aufnahme bzw. einem Einschub eines Terminals zum Auslesen der elektronischen Gesundheitskarte. Beispielsweise kann am Rand in der Aufnahme ein Sensor an der Stelle angeordnet sein, an welcher sich bei einem Anordnen der elektronischen Gesundheitskarte in der Aufnahme der markierte Abschnitt befinden sollte. Ist der markierte Abschnitt vorhanden, wird der Sensor betätigt und das Terminal erfasst das Vorhandensein des markierten Abschnitts, womit eine Zustimmung zu einer Organspende signalisiert wird. Fehlt der markierte Abschnitt, wird der Sensor nicht betätigt und das Terminal erfasst das Fehlen des markierten Abschnitts, womit eine Ablehnung zu einer Organspende signalisiert wird. Beispielsweise ist in der Aufnahme bzw. Einschub ein weiterer Sensor zum Erfassen der Gegenwart der elektronischen Gesundheitskarte vorgesehen, unabhängig von einem Vorhandensein oder Fehlen des markierten Abschnitts.

Nach Ausführungsformen handelt es sich bei dem markierten Abschnitt um einen Eckabschnitt des Kartenkörpers. Ausführungsformen können den Vorteil haben, dass ein Eckabschnitt leicht abtrennbar ist und sein Fehlen, insbesondere maschinell mittels eines Sensors, leicht erfassbar ist.

Nach Ausführungsformen resultiert aus einem Entfernen des markierten Abschnitts eine Aussparung in einem Rand des Kartenkörpers.

Nach Ausführungsformen ist der markierte Abschnitt vollständig von der Sollbruchlinie umgrenzt. Ausführungsformen können den Vorteil haben, dass der markierte Abschnitt in diesem Fall innerhalb des Kartenkörpers angeordnet ist, wodurch der markierte Abschnitt effektiv vor einem versehentlichen Abtrennen geschützt werden kann. Nach Ausführungsformen ist der markierte Abschnitt in einem zentralen Bereich des Kartenkörpers angeordnet.

Nach Ausführungsformen resultiert aus einem Entfernen des markierten Abschnitts eine Ausnehmung bzw. einem Loch in dem Kartenkörper. Ausführungsformen können den Vorteil haben, dass ein Fehlen des markierten Abschnitts, insbesondere maschinell mittels eines Sensors, leicht erfassbar ist.

Nach Ausführungsformen umfasst die Gesundheitskarte eine Leiterschleife zum Erfassen, ob der markierte Abschnitt vorhanden ist. Die Leiterschleife erstreckt sich durch den markierten Abschnitt, sodass die Leiterschleife bei einem Entfernen des markierten Abschnitts unterbrochen wird. Ausführungsformen können den Vorteil haben, dass die Leiterschleife es der Gesundheitskarte, beispielsweise einem Prozessor der Gesundheitskarte, ermöglicht zu bestimmen, ob der markierte Abschnitt vorhanden ist oder nicht. Nach Ausführungsformen ist die Leiterschleife elektrisch leitend mit dem Prozessor verbunden ist. Der Prozessor ist konfiguriert zu prüfen, ob die Leiterschleife unterbrochen ist.

Die Leiterschleife ist beispielsweise mit dem Prozessor der Gesundheitskarte elektrisch leitend verbunden. Der Prozessor wird mit Hilfe der Leiterschleife in die Lage versetzt ein Vorhandensein oder Fehlen des markierten Abschnitts zu erfassen. Ist die Leiterschleife bzw. deren elektrische Leitfähigkeit unterbrochen, so fehlt der markierte Abschnitt. Ist die Leiterschleife bzw. deren elektrische Leitfähigkeit nicht unterbrochen, so ist der markierte Abschnitt vorhanden. Die elektrische Leitfähigkeit der Leiterschleife kann der Prozessor beispielsweise dadurch testen, dass er versucht einen Strom durch diese zu schicken. Ist der Prozessor damit erfolgreich, ist die Leiterschleife ununterbrochen. Ist der Prozessor damit nicht erfolgreich, so ist die Leiterschleife unterbrochen.

Diesen Test kann die Gesundheitskarte bzw. deren Prozessor beispielsweise bei Bedarf, etwa auf Anfrage hin, etwa auf Anfrage eines Terminals hin, ausführen. Beispielsweise der Prozessor das Ergebnis des Tests zum Antworten auf die Anfrage. Beispielsweise umfasst eine Antwort die Information, dass der markierte Abschnitt fehlt bzw. vorhanden ist. Dies kann beispielsweise durch ein einzelnes Bit in einer Antwort gemäß einem vorgegebenen Format angezeigt werden. Beispielsweise speichert die Gesundheitskarte das Ergebnis des Tests ab. Beispielsweise wird der Test nur unter der Voraussetzung ausgeführt, dass eine in dem Speicher der Gesundheitskarte umfasste Information anzeigt, dass der markierte Abschnitt vorhanden ist bzw. die bisherigen Tests ein Vorhandensein des markierten Abschnitts angezeigt haben. Ergibt ein Test, dass der markierte Abschnitt fehlt, wird diese Information von der Gesundheitskarte abgespeichert, beispielsweise durch Setzen einer entsprechenden Flag. Beispielsweise wird die entsprechende Information in einem geschützten Speicherbereich des Speichers der Gesundheitskarte abgespeichert. Inertial, d.h. in einem Ausgabezustand der Gesundheitskarte, ist in der Gesundheitskarte beispielsweise keine Information bezüglich des Vorhandenseins oder Fehlens des markierten Abschnitts gespeichert. Inertial, d.h. in dem Ausgabezustand der Gesundheitskarte, ist in der Gesundheitskarte beispielsweise die Information gespeichert, dass der markierte Abschnitt vorhanden ist.

Wurde infolge eines Tests der Leiterschleife die Information gespeichert, dass der markierte Abschnitt fehlt, ergeben zukünftige Prüfungen des Speichers der Gesundheitskarte somit jeweils, dass eine Information vorhabenden ist, welche das Fehlen des markierten Abschnitts anzeigt, beispielsweise in Form des gesetzten Flags. Die Gesundheitskarte führt in diesem Fall beispielsweise keinen Test der Leiterschleife mehr durch, da der einmal entfernte markierte Abschnitt nicht dazu vorgesehen ist wieder hinzugefügt oder ersetzt zu werden. Beispielsweise erstellt die Gesundheitsfrage im Falle einer Anfrage dann eine Antwort basierend auf dem Ergebnis der Speicherabfrage mit der Information, dass der markierte Bereich fehlt.

Nach Ausführungsformen ist der Prozessor der elektronischen Gesundheitskarte ferner zu Folgendem konfiguriert:
Empfangen einer Abfrage eines Organspenderstatus des Inhabers der elektronischen Gesundheitskarte von dem Terminal über eine Kommunikationsschnittstelle der Gesundheitskarte,
in Antwort auf die empfangene Abfrage, Prüfen, ob die Leiterschleife unterbrochen ist,
Erzeugen einer Antwort auf die empfangene Abfrage, welche das Ergebnis der Prüfung umfasst, wobei das Ergebnis der Prüfung den Organspenderstatus des Inhabers der elektronischen Gesundheitskarte anzeigt,
in Antwort auf die empfangene Abfrage, Senden der erzeugten Antwort an das Terminal über die Kommunikationsschnittstelle der Gesundheitskarte.

Nach Ausführungsformen ist der Prozessor der elektronischen Gesundheitskarte ferner zu Folgendem konfiguriert:
Empfangen einer Abfrage eines Organspenderstatus des Inhabers der elektronischen Gesundheitskarte von dem Terminal über eine Kommunikationsschnittstelle der Gesundheitskarte,
in Antwort auf die empfangene Abfrage, Abfragen eines in einem Speicher der Gesundheitskarte gespeicherten Prüfungsergebnisse einer Prüfung des Prozessors, ob die Leiterschleife unterbrochen ist,
falls das gespeicherte Prüfungsergebnis eine Unterbrechung der Leiterschleife anzeigt, verwenden des gespeicherten Prüfungsergebnisses, falls das gespeicherte Prüfungsergebnis keine Unterbrechung der Leiterschleife anzeigt, erneutes Prüfen, ob die Leiterschleife unterbrochen ist, wobei das gespeicherte Prüfungsergebnis durch das Prüfungsergebnis der erneuten Prüfung ersetzt und das Prüfungsergebnis der erneuten Prüfung verwendet wird,
Erzeugen einer Antwort auf die empfangene Abfrage, welche das Ergebnis der Prüfung umfasst, wobei das Ergebnis der Prüfung den Organspenderstatus des Inhabers der elektronischen Gesundheitskarte anzeigt,
in Antwort auf die empfangene Abfrage, Senden der erzeugten Antwort an das Terminal über die Kommunikationsschnittstelle der Gesundheitskarte.

Beispielsweise sendet ein Terminal eine Anfrage an die Gesundheitskarte, beispielsweise über ein kontaktlose oder kontaktbasierte Kommunikationsschnittstelle der Gesundheitskarte. Auf den Empfang der Anfrage hin, testet die Gesundheitskarte bzw. der Prozessor der Gesundheitskarte die elektrische Leitfähigkeit der Leiterschleife. Der Prozessor erfasst ein Fehlen des markierten Abschnitts beispielsweise anhand einer Unterbrechung der Leiterschleife bzw. deren elektrischer Leitfähigkeit, während der Prozessor ein Vorhandensein des markierten Abschnitts beispielsweise daran erkennt, dass die Leiterschleife bzw. deren elektrische Leitfähigkeit nicht unterbrochen ist. Das Ergebnis des Tests sendet die Gesundheitskarte in Antwort auf die Anfrage des Terminals, beispielsweise über ihre kontaktlose oder kontaktbasierte Kommunikationsschnittstelle, an das Terminal.

Beispielsweise erstellt die Gesundheitsfrage im Falle einer Anfrage eine Antwort basierend auf dem Ergebnis der Speicherabfrage mit der Information, dass der markierte Bereich fehlt, ohne dass ein in Folge der Abfrage zusätzlich initiierter Test der Leiterschleife erfolgt. Dies ist beispielsweise der Fall, wenn die Speicherabfrage ergibt, dass der markierte Abschnitt fehlt.

Beispielsweise ist die Antwort der Gesundheitskarte mit einem kryptographischen Signaturschlüssel der Gesundheitskarte. Beispielsweis ist ein entsprechender Signaturschlüssel der Gesundheitskarte in einem Speicher der Gesundheitskarte, insbesondere in einem geschützten Speicherbereich eines Speichers der Gesundheitskarte, gespeichert. Ferner erfolgt die Kommunikation zwischen der Gesundheitskarte und dem Terminal beispielsweise über eine verschlüsselte Kommunikationsverbindung, insbesondere über eine mittels einer Ende-zu-Ende-Verschlüsselung verschlüsselten Kommunikationsverbindung. Beispielsweise ist die Kommunikationsverbindung symmetrisch und/oder asymmetrisch verschlüsselt.

Nach Ausführungsformen ist eine Voraussetzung für das Antworten auf die Abfrage des Organspenderstatus durch die elektronische Gesundheitskarte ein erfolgreicher Nachweis einer Berechtigung zum Abfragen des Organspenderstatus durch das Terminal gegenüber der elektronischen Gesundheitskarte ist. Ausführungsformen können den Vorteil haben, dass Informationen zu der Organspendebereitschaft des Inhabers der elektronischen Gesundheitskarte nur an berechtigte Terminals übertragen wird. Eine entsprechende Berechtigung kann beispielsweise mittels eines Berechtigungszertifikats nachgewiesen werden.

Nach Ausführungsformen ist die Kommunikationsschnittstelle der elektronischen Gesundheitskarte zu einer kontaktbasierten Kommunikation mit einem Terminal konfiguriert. Nach Ausführungsformen ist die Kommunikationsschnittstelle der Gesundheitskarte also eine kontaktbasierte Kommunikationsschnittstelle. Beispielsweise wird die elektronische Gesundheitskarte zu diesem Zweck in eine Aufnahme bzw. einen Einschub des Terminals eingeschoben.

Nach Ausführungsformen umfasst der markierte Abschnitt eine Schaltung. Die Schaltung umfasst eine Antenne und ein mit der Antenne elektrisch leitend verschaltetes Leuchtmittel. Die Schaltung ist dazu konfiguriert, das Leuchtmittel mittels einer induktiven Energieeinkopplung elektromagnetischer Energie in die Antenne zum Leuchten zu bringen.

Ausführungsformen können den Vorteil haben, dass durch induktive Energieeinkopplung elektromagnetischer Energie in die Antenne das Leuchtmittel, beispielsweise eine Diode, zum Leuchten gebracht werden kann. Durch Einkoppeln elektromagnetischer Energie in die Antenne des Leuchtmittels kann mithin in einfacher Weise überprüft werden, ob der markierte Abschnitt noch vorhanden ist. Ist der markierte Abschnitt vorhanden, ist das leuchtende Leuchtmittel vorhanden. Fehlt der markierte Abschnitt, fehlt das Leuchtmittel und es kann kein Leuchten erfasst werden. Eine Energieeinkopplung kann beispielsweise durch ein Terminal im Zuge einer Prüfung der Gesundheitskarte erfolgen.

Beispielsweise umfasst der entfernbare markierte Abschnitt eine gewickelte Antenne und ein damit verschaltetes Leuchtmittel. Beispielsweise in einem RFID-Feld leuchtet das Leuchtmittel aufgrund der eingebrachten elektromagnetischen Energie, wenn der markierte Abschnitt beibehalten wurde. Wenn er entfernt wurde, tritt das Leuchten nicht auf, so dass eine rasche Indikation gewährleistet ist.

Nach Ausführungsformen kann der markierte Abschnitt von der Schaltung beispielsweise auch nur eines der folgenden Elemente umfassen: einen Teil der Antenne, die vollständige Antenne, das Leuchtmittel, eine elektrisch leitende Verbindung zwischen Antenne und Leuchtmittel.

Nach Ausführungsformen verwendet die Gesundheitskarte die kontaktbasierte Kommunikationsschnittstelle dazu, dem Terminal mitzuteilen, ob der markierte Abschnitt vorhanden ist oder fehlt. Diese Information basiert beispielsweise auf einem Test der Leiterschleife bzw. deren elektrische Leitfähigkeit durch den Prozessor der Gesundheitskarte.

Nach Ausführungsformen ist die Kommunikationsschnittstelle der Gesundheitskarte eine kontaktlose Kommunikationsschnittstelle. Nach Ausführungsformen umfasst die Kommunikationsschnittstelle eine oder mehrere Antennen und ist zu einer kontaktlosen Kommunikation mit einem Terminal konfiguriert.

Nach Ausführungsformen verwendet die Gesundheitskarte die kontaktlose Kommunikationsschnittstelle dazu, dem Terminal mitzuteilen, ob der markierte Abschnitt vorhanden ist oder fehlt. Diese Information basiert beispielsweise auf einem Test der Leiterschleife bzw. deren elektrische Leitfähigkeit durch den Prozessor der Gesundheitskarte.

Nach Ausführungsformen sind die Antennenführung der ein oder mehreren Antennen und eine Positionierung des markierten Abschnitts so aufeinander abgestimmt, dass sich keine der Antennen durch den markierten Abschnitt erstreckt. Ausführungsformen können den Vorteil haben, dass durch ein Abtrennen des markierten Abschnitts die Antennen und mithin die kontaktlose Kommunikation der elektronischen Gesundheitskarte mit dem Terminal nicht beeinträchtigt wird.

Nach Ausführungsformen umfasst die Kommunikationsschnittstelle genau eine Antenne, welche sich durch den markierten Abschnitt erstreckt. Ausführungsformen können den Vorteil haben, dass durch ein Abtrennen des markierten Abschnitts dauerhaft die Antenne unterbrochen und somit die kontaktlose Kommunikation der der elektronischen Gesundheitskarte mit dem Terminal unterbunden wird. In diesem Fall ist anhand der Fähigkeit der elektronischen Gesundheitskarte zur kontaktlosen Kommunikation der Zustand des markierten Abschnitts ableitbar. Ist die elektronische Gesundheitskarte zu einer kontaktlosen Kommunikation mit dem Terminal fähig, können die elektronischen Gesundheitskarte und das Terminal daran erkennen, dass der markierte Abschnitt vorhanden ist und eine Zustimmung zu einer Organspende besteht. Ist die elektronische Gesundheitskarte zu keiner kontaktlosen Kommunikation mit dem Terminal fähig, können die elektronischen Gesundheitskarte und das Terminal daran erkennen, dass der markierte Abschnitt fehlt und eine Organspende abgelehnt wird.

Nach Ausführungsformen ist die Kommunikationsschnittstelle ferner zu einer kontaktbasierten Kommunikation mit einem Terminal konfiguriert. Durch ein Fehlschlagen der kontaktlosen Kommunikation mit dem Terminal infolge eines Fehlens des markierten Abschnitts, während die kontaktbasierte Kommunikation erfolgreich ist, wird dem Terminal gegenüber eine Ablehnung des Inhabers zu der Organspende signalisiert. Ausführungsformen können den Vorteil haben, dass durch die kontaktbasierte Kommunikation die Anwesenheit der elektronischen Gesundheitskarte, etwa in einer Aufnahme bzw. einem Einschub des Terminals, seitens des Terminals detektiert werden kann. Ist die elektronische Gesundheitskarte anwesend, aber ein kontaktloser Kommunikationsaufbau scheitert im Gegensatz zu einem kontaktbasierten Kommunikationsaufbau, so weiß das Terminal, dass die Antenne zur kontaktlosen Kommunikation unterbrochen ist, der markierte Abschnitt mithin fehlt und daher eine Organspende seitens des Inhabers der elektronischen Gesundheitskarte abgelehnt wird.

Nach Ausführungsformen umfasst die Kommunikationsschnittstelle zumindest zwei Antenne von denen sich eine erste Antenne durch den markierten Abschnitt erstreckt, während sich eine zweite Antenne nicht durch den markierten Abschnitt erstreckt. Ausführungsformen können den Vorteil haben, dass abhängig von dem Vorhandensein oder Fehlen des markierten Abschnitts beide oder nur eine der beiden Antennen der elektronischen Gesundheitskarte funktionstüchtig sind. Indem erfasst wird, ob beide oder nur eine der beiden Antennen funktionsfähig sind, kann durch die elektronische Gesundheitskarte und/oder das Terminal erfasst werden, ob der markierte Abschnitt vorhanden ist oder nicht.

Nach Ausführungsformen sind die beiden Antennen für eine induktive Energieeinkopplung elektromagnetischer Energie des Terminals in die Gesundheitskarte konfiguriert. Der Prozessor ist dazu konfiguriert einen relativen Abfall der eingekoppelten Energie der ersten Antenne gegenüber der zweiten Antenne zu erfassen. Der Prozessor ist ferner dazu konfiguriert, anhand eines Überschreitens eines ersten vordefinierten Schwellenwerts durch den erfassten relativen Abfall ein Fehlen des markierten Abschnitts zu detektieren. Der Prozessor sendet auf ein Detektieren des Fehlens des markierten Abschnitts hin ein Ablehnungssignal erzeugt und an das Terminal. Das Ablehnungssignal zeigt eine Ablehnung des Inhabers zu der Organspende an.

Ausführungsformen können den Vorteil haben, dass eine Energieversorgung der elektronischen Gesundheitskarte mittels Energy Harvesting ermöglicht wird. Zugleich kann anhand der über die beiden Antennen eingekoppelten Energie die Funktionsfähigkeit der Antennen und mithin das Vorhandensein oder Fehlen des markierten Abschnitts erfasst werden. Wird über eine der beiden Antenne kein oder kaum noch Energie mehr eingekoppelt, so ist diese unterbrochen und es fehlt mithin der markierte Abschnitt. Dieses Fehlen des markierten Abschnitts stellt eine Ablehnung einer Organspende dar, welche dem Terminal beispielsweise mittels eines Ablehnungssignal angezeigt werden kann, welches beispielsweise über die funktionierende Antenne an das Terminal geschickt werden kann. Nach Ausführungsformen erfolgt das Senden des Ablehnungssignals über die erste Antenne.

Nach Ausführungsformen ist eine Voraussetzung für das Erzeugen und Senden des Ablehnungssignals, ein Empfang einer Abfrage der Organspenderstatus des Inhabers der elektronischen Gesundheitskarte von dem Terminal.

Nach Ausführungsformen ist der Prozessor ferner dazu konfiguriert ist, falls kein relativer Abfall der eingekoppelten Energie der ersten Antenne gegenüber der zweiten Antenne oder ein relativer Abfall erfasst wird, welcher den ersten vordefinierten Schwellenwert nicht überschreitet, ein Vorhandensein des markierten Abschnitts zu detektieren. Auf ein Detektieren des Vorhandenseins des markierten Abschnitts, erzeugt der Prozessor ein Zustimmungssignal, welches eine Zustimmung des Inhabers zu der Organspende anzeigt, und sendet das Zustimmungssignal an das Terminal. Nach Ausführungsformen erfolgt das Senden des Zustimmungssignals über die erste oder zweite Antenne.

Wird über beide Antennen Energie in einem für den Regelbetrieb zu erwartenden Umfang eingekoppelt, so ist keine der Antennen unterbrochen und der markierte Abschnitt mithin vorhanden. Dieses Vorhandensein des markierten Abschnitts stellt eine Zustimmung zu einer Organspende dar, welche dem Terminal beispielsweise mittels eines Zustimmungssignal angezeigt werden kann.

Nach Ausführungsformen ist eine Voraussetzung für das Erzeugen und Senden des Zustimmungssignals, ein Empfang einer Abfrage der Organspenderstatus des Inhabers der elektronischen Gesundheitskarte von dem Terminal.

Nach Ausführungsformen ist die elektronische Gesundheitskarte ferner dazu konfiguriert unter Verwendung der beiden Antennen eine vordefinierte Energiemenge abstrahlen. Ein Abfall der abgestrahlten Energie infolge einer Unterbrechung der ersten Antenne bei einer Anordnung der elektronischen Gesundheitskarte an einer vordefinierten Position relative zu dem Terminal, welcher in einem Abfall des von dem Terminal empfangbaren Teils der abgestrahlten Energie unter einen zweiten vordefinierten Schwellenwert resultiert, zeigt gegenüber dem Terminal ein Fehlen des markierten Abschnitts an.

Ausführungsformen können den Vorteil haben, dass das Terminal anhand der Energiemenge, welche es von der elektronischen Gesundheitskarte, die an einer vordefinierten Position relative zu dem Terminal angeordnet ist, empfängt bestimmen kann, ob beide Antennen der elektronischen Gesundheitskarte zur Energieübertragung beitragen der nur eine der beiden. Somit kann das Terminal anhand der Energiemenge detektieren, ob der markierte Abschnitt vorhanden ist oder fehlt.

Ist beispielsweise die elektronische Gesundheitskarte an der vordefinierten Position relative zu dem Terminal angeordnet und strahlt sie ohne eine Unterbrechung der ersten Antenne unter Verwendung beider Antennen die vordefinierte Energiemenge ab, so resultiert diese vordefinierte Energiemenge in einem von dem Terminal empfangbaren Teil der abgestrahlten Energie, welcher den zweiten vordefinierten Schwellenwert nicht unterschreitet. Ein entsprechendes Nichtunterschreiten des von dem Terminal empfangbaren Teil der abgestrahlten Energie zeigt gegenüber dem Terminal ein Vorhandensein des markierten Abschnitts an.

Nach Ausführungsformen wird die vordefinierte Position relative zu dem Terminal beispielsweise durch eine für ein Auflegen der elektronischen Gesundheitskarte vorgesehenen Auflagefläche des Terminals definiert. Nach Ausführungsformen wird die vordefinierte Position relative zu dem Terminal beispielsweise durch eine Aufnahme bzw. einen Einschub des Terminals definiert, welche dazu konfiguriert ist die elektronische Gesundheitskarte aufzunehmen. Zum Anordnen der elektronischen Gesundheitskarte an der vordefinierten Position, wird diese beispielsweise in die Aufnahme des Terminals eingeschoben.

Nach Ausführungsformen ist der Prozessor der elektronischen Gesundheitskarte ferner zu Folgendem konfiguriert:
Empfangen einer Abfrage eines Organspenderstatus des Inhabers der elektronischen Gesundheitskarte von dem Terminal über die zweite Antenne,
in Antwort auf die empfangene Abfrage, Erzeugen eines ersten vordefinierten Signals,
Initiieren eines Sendens des vordefinierten Signals an das Terminal über die zweite Antenne, wobei das erste vordefinierte Signal eine Zustimmung des Inhabers zu einer Organspende signalisiert.

Ausführungsformen können den Vorteil haben, dass das Terminal daran, ob es auf das Senden der Abfrage des Organspenderstatus des Inhabers der elektronischen Gesundheitskarte das erste vordefinierte Signals empfängt, bestimmen kann, ob der markierte Abschnitt vorhanden ist oder nicht. Empfängt das Terminal das erste vordefinierte Signal, signalisiert dieser Empfang dem Terminal ein Funktionieren der ersten Antenne, was ein Vorhandensein des markierten Abschnitts und mithin eine Zustimmung des Inhabers zu einer Organspende. Bleibt das erste vordefinierte Signal aus, signalisiert dieses Ausbleiben des ersten Signals dem Terminal ein Fehlen des markierten Abschnitts und mithin eine Ablehnung des Inhabers der Organspende.

Nach Ausführungsformen ist der Prozessor der elektronischen Gesundheitskarte ferner zu Folgendem konfiguriert:
in Antwort auf die empfangene Abfrage, Erzeugen eines zweiten vordefinierten Signals,
Senden des zweiten vordefinierten Signals an das Terminal über die zweite Antenne, wobei das zweite vordefinierte Signal eine Bestätigung des Empfangens der Abfrage durch die elektronische Gesundheitskarte signalisiert.

Ausführungsformen können den Vorteil haben, dass das zweite Signal dem Terminal gegenüber den Empfang der Abfrage bestätigt. Das Terminal kann anhand des zweiten Signals mithin detektieren, dass die elektronische Gesundheitskarte anwesend ist und eine kontaktlose Kommunikation funktioniert. Bleibt das erste Signal mithin als Antwort auf die Abfrage aus, während das zweite Signal empfangen wir, weiß das Terminal, dass die kontaktlose Kommunikation funktioniert, aber der markierte Abschnitt fehlt und eine Organspende abgelehnt wird.

Nach Ausführungsformen ist eine Voraussetzung für das Antworten auf die Abfrage des Organspenderstatus durch die elektronische Gesundheitskarte ein erfolgreicher Nachweis einer Berechtigung zum Abfragen des Organspenderstatus durch das Terminal gegenüber der elektronischen Gesundheitskarte ist. Ausführungsformen können den Vorteil haben, dass Informationen zu der Organspendebereitschaft des Inhabers der elektronischen Gesundheitskarte nur an berechtigte Terminals übertragen wird. Eine entsprechende Berechtigung kann beispielsweise mittels eines Berechtigungszertifikats nachgewiesen werden.

Nach Ausführungsformen ist eine Voraussetzung für das Bereitstellen der Daten zum Auslesen durch das Terminal ein erfolgreicher Nachweis einer Berechtigung zum Auslesen der Daten durch das Terminal gegenüber der elektronischen Gesundheitskarte.

Ausführungsformen können den Vorteil haben, dass in der elektronischen Gesundheitskarte gespeicherte Daten des Inhabers der elektronischen Gesundheitskarte nur an berechtigte Terminals übertragen werden. Eine entsprechende Berechtigung kann beispielsweise mittels eines Berechtigungszertifikats nachgewiesen werden.

Nach Ausführungsformen können die in der Gesundheitskarte gespeicherten personenbezogenen und krankenversicherungsbezogenen Daten des Inhabers der elektronischen Gesundheitskarte beispielsweise zusätzlich oder in Ergänzung zu der Deklaration des Willens des Inhabers der elektronischen Gesundheitskarte bezüglich der Bereitschaft zu einer Organ- oder Gewebespende mittels des markierten Abschnitts die Erklärung der Zustimmung oder der Ablehnung der Bereitschaft zu einer Organ- oder Gewebespende auch in elektronsicher Form umfassen. Beispielsweise ist die Erklärung der Zustimmung oder der Ablehnung der Bereitschaft zu einer Organ- oder Gewebespende in verschlüsselter Form in dem Speicher gespeichert.

Ausführungsformen umfassen ferner ein Terminal, welches einen Prozessor, einen Speicher mit Programminstruktionen und eine Kommunikationsschnittstelle umfasst. Der Prozessor ist dazu konfiguriert, in einer Gesundheitskarte gespeicherte personenbezogene und krankenversicherungsbezogene Daten eines Inhabers der elektronischen Gesundheitskarte unter Verwendung der Programminstruktionen über die Kommunikationsschnittstelle auszulesen.

Das Terminal ist ferner konfiguriert zum Erfassen, ob ein Abschnitt des Kartenkörpers der elektronischen Gesundheitskarte vorhanden ist, dessen Vorhandensein eine Zustimmung des Inhabers zu einer Organspende anzeigt. Ein Fehlen des Abschnitts zeigt eine Ablehnung des Inhabers zu der Organspende an.

Ausführungsformen können den Vorteil haben, dass mittels des Terminals elektronisch und automatisch erfasst werden kann, ob der Inhaber der elektronischen Gesundheitskarte einer Organspende zustimmt, oder diese ablehnt.

Nach Ausführungsformen umfasst das Terminal eine Aufnahme, welche zum Aufnehmen der elektronischen Gesundheitskarte konfiguriert ist. In der Aufnahme ist ein Sensor angeordnet ist, welcher dazu konfiguriert ist, das Vorhandensein des Abschnitts des Kartenkörpers der elektronischen Gesundheitskarte zu erfassen. Ausführungsformen können den Vorteil haben, dass der Sensor erfassen kann, ob der markierte Abschnitt vorhanden ist oder nicht und mithin, ob eine Zustimmung oder Ablehnung zu Organspenden vorliegt.

Nach Ausführungsformen zeigt ein Betätigen des Sensors durch in der Aufnahme angeordneten Kartenkörper ein Vorhandensein des Abschnitts an. Der Sensor ist beispielsweise an einer Stelle in der Aufnahme angeordnet, an welcher sich der markierte Abschnitt befinden sollte, wenn die elektronische Gesundheitskarte ein der Aufnahme angeordnet ist.

Nach Ausführungsformen ist die Kommunikationsschnittstelle des Terminals zu einer kontaktlosen Kommunikation mit der elektronischen Gesundheitskarte konfiguriert.

Nach Ausführungsformen ist die Kommunikationsschnittstelle des Terminals ferner zu einer kontaktbasierten Kommunikation mit der elektronischen Gesundheitskarte konfiguriert. Der Prozessor des Terminals ist ferner zu Folgendem konfiguriert:
Ausführen eines ersten Versuchs eines kontaktlosen Aufnehmens einer Kommunikation mit der elektronischen Gesundheitskarte und eines zweiten Versuchs eines kontaktbasierten Aufnehmens einer Kommunikation mit der elektronischen Gesundheitskarte,
falls der erste Versuch fehlschlägt, während der zweite Versuch erfolgreich ist, Erzeugen eines Ablehnungsvermerks, dass eine Ablehnung des Inhabers zu der Organspende vorliegt.

Ausführungsformen können den Vorteil haben, dass anhand des Fehlschlags der kontaktlosen Kommunikation auf eine Unterbrechung einer Antenne der elektronischen Gesundheitskarte infolge eines Entfernens des markierten Abschnitts geschlossen werden kann. Dies bedeutet eine Ablehnung einer Organspende durch den Inhaber der elektronischen Gesundheitskarte.

Falls der erste Versuch erfolgreich ist wird beispielsweise ein Zustimmungsvermerk erzeugt, dass eine Zustimmung des Inhabers zu der Organspende vorliegt. Funktioniert die kontaktlose Kommunikation, ist der markierte Abschnitt vorhanden.

Nach Ausführungsformen ist das Terminals ferner zu einem induktiven Energieeinkoppeln elektromagnetischer Energie in die elektronische Gesundheitskarte konfiguriert. Der Prozessor des Terminals ist ferner konfiguriert zu einem Empfangen eines Ablehnungssignals, welches eine Ablehnung des Inhabers der elektronischen Gesundheitskarte zu der Organspende anzeigt, und zu einem Empfangen eines Zustimmungssignals, welches eine Zustimmung des Inhabers der elektronischen Gesundheitskarte zu der Organspende anzeigt.

Ausführungsformen können den Vorteil haben, dass das Terminal die elektronische Gesundheitskarte mittel Energy Harvesting mit Energie versorgen kann. Zugleich kann die elektronische Gesundheitskarte anhand der eingekoppelten Energie auf die Funktionsfähigkeit der dazu verwendeten Antennen und mithin auf ein Vorhandensein oder Fehlend es markierten Abschnitts schließen. In Abhängigkeit davon, ob der markierte Abschnitt vorhanden ist oder nicht wird das Zustimmungssignal bzw. das Ablehnungssignal erzeigt und an das Terminal gesendet.

Nach Ausführungsformen ist der Prozessor des Terminals ferner konfiguriert zu einem Erzeugen einer Abfrage des Organspenderstatus eines Inhabers der elektronischen Gesundheitskarte und zu einem Senden der Abfrage an die elektronische Gesundheitskarte unter Verwendung der Kommunikationsschnittstelle des Terminals. Bei dem Ablehnungssignal und dem Zustimmungssignal handelt es sich jeweils um eine Antwort auf eine Abfrage des Organspenderstatus durch das Terminal.

Nach Ausführungsformen ist der Prozessor des Terminals ferner zu Folgendem konfiguriert ist:
Erzeugen einer Abfrage des Organspenderstatus eines Inhabers der elektronischen Gesundheitskarte,
Senden der Abfrage an die elektronische Gesundheitskarte unter Verwendung der Kommunikationsschnittstelle des Terminals, wobei ein Empfangen eines ersten vordefinierten Signals von der elektronischen Gesundheitskarte in Antwort auf das Senden der Abfrage ein Vorhandensein des markierten Abschnitts anzeigt.

Ausführungsformen können den Vorteil haben, dass das erste vordefinierte Signal über eine Antenne gesendet wird, welche sich durch den markierten Abschnitt erstreckt. Mithin signalisiert ein Empfang des ersten vordefinierten Signals eine Funktionsfähigkeit der entsprechenden Antenne. Mithin ist der markierte Abschnitt vorhanden und eine Zustimmung zu der Organspende liegt vor. Bleibt das erste vordefinierte Signal aus, ist die Antenne unterbrochen, der markierte Abschnitt fehlt und eine Organspende wird abgelehnt.

Nach Ausführungsformen bestätigt ein Empfangen eines zweiten vordefinierten Signals von der elektronischen Gesundheitskarte in Antwort auf das Senden der Abfrage den gesundheitskartenseitigen Empfang der Abfrage. Ausführungsformen können den Vorteil haben, dass durch das zweite vordefinierte Signal, welches über eine Antenne gesendet wird, die sich nicht durch den markierten Abschnitt erstreckt und mithin von diesem unabhängig ist, ein Empfang der Abfrage des Organspenderstatus eines Inhabers der elektronischen Gesundheitskarte durch die elektronische Gesundheitskarte bestätigt wird.

Nach Ausführungsformen ist der Prozessor des Terminals ferner zu Folgendem konfiguriert:
unter Verwendung der Kommunikationsschnittstelle des Terminals Bestimmen eine Signalstärke, mit welcher Signale der elektronischen Gesundheitskarte im Zuge einer kontaktlosen Kommunikation von dem Terminal empfangen werden, wobei die elektronische Gesundheitskarte an einer vordefinierten Position relativ zu dem Terminal angeordnet ist, und,
falls die Signalstärke einen vordefinierten Schwellenwert unterschreitet, Erzeugen eines Ablehnungsvermerks, dass eine Ablehnung des Inhabers zu der Organspende vorliegt.

Ausführungsformen können den Vorteil haben, dass anhand der Signalstärke bestimmt werden kann, ob die Antennen der elektronischen Gesundheitskarte vollfunktionsfähig sind. Daraus ergibt sich, ob der markierte Abschnitt vorhanden ist oder nicht bzw. ob eine Zustimmung oder Ablehnung zu der Organspende vorliegt.

Nach Ausführungsformen ist der Prozessor des Terminals dazu ferner konfiguriert, einen Zustimmungsvermerk zu erzeugt, dass eine Zustimmung des Inhabers zu der Organspende vorliegt, falls die Signalstärke den vordefinierten Schwellenwert nicht unterschreitet.

Nach Ausführungsformen definiert das Terminal die Position, an welcher die elektronische Gesundheitskarte zur Bestimmung der Signalstärke anzuordnen ist. Beispielsweise wird die Position durch eine äußere Fläche eines Gehäuses des Terminals definiert, auf bzw. an welche die elektronische Gesundheitskarte zur Bestimmung der Signalstärke auf bzw. anzulegen ist. Beispielsweise wird die Position durch eine Aufnahme des Terminals zum Aufnehmen der elektronischen Gesundheitskarte definiert, wobei die elektronische Gesundheitskarte zur Bestimmung der Signalstärke in der Aufnahme anzuordnen ist.

Nach Ausführungsformen ist der Prozessor des Terminals ferner zu Folgendem konfiguriert:
Erzeugen einer Abfrage des Organspenderstatus eines Inhabers der elektronischen Gesundheitska rte,
Senden der Abfrage an die elektronische Gesundheitskarte unter Verwendung der Kommunikationsschnittstelle des Terminals,
Empfangen einer Antwort der elektronischen Gesundheitskarte, welche das Ergebnis einer Prüfung der elektronischen Gesundheitskarte umfasst, ob eine sich durch den markierten Abschnitt des Kartenkörpers der Gesundheitskarte erstreckende Leiterschleife unterbrochen ist, wobei das Ergebnis der Prüfung anzeigt, ob der Abschnitt der elektronischen Gesundheitskarte vorhanden ist.

Ausführungsformen können den Vorteil haben, dass das Terminal anhand der Antwort der Gesundheitskarte erfassen kann, ob der Abschnitt des Kartenkörpers der Gesundheitskarte vorhanden ist. Die Antwort der Gesundheitskarte basiert dabei auf einer Prüfung der Leiterschleife durch die Gesundheitskarte bzw. deren Prozessor. Somit kann diese Prüfung durch die Gesundheitskarte selbständig und automatisiert ausgeführt werden, wobei sich das Terminal diese Prüffunktionalität der Gesundheitskarte zu nutzen macht. Ausführungsformen umfassen ferner ein System, welches eine elektronische Gesundheitskarte nach einer der zuvor beschriebenen Ausführungsformen sowie ein Terminal nach der zuvor beschriebenen Ausführungsformen umfasst.

Im Weiteren werden Ausführungsformen der Erfindung mit Bezugnahme auf die Zeichnungen näher erläutert. Es zeigen:
- Figur 1: ein schematisches Diagramm einer exemplarischen Gesundheitskarte,
- Figur 2: ein schematisches Diagramm einer exemplarischen Gesundheitskarte,
- Figur 3: schematische Querschnitte exemplarischen Gesundheitskarte,
- Figur 4: schematische Querschnitte exemplarischen Gesundheitskarte,
- Figur 5: schematische Querschnitte exemplarischen Gesundheitskarte,
- Figur 6: ein schematisches Diagramm einer exemplarischen Gesundheitskarte,
- Figur 7: ein schematisches Diagramm einer exemplarischen Gesundheitskarte,
- Figur 8: ein schematisches Diagramm einer exemplarischen Gesundheitskarte,
- Figur 9: ein schematisches Diagramm einer exemplarischen Gesundheitskarte,
- Figur 10: ein schematisches Diagramm einer exemplarischen Gesundheitskarte,
- Figur 11: ein schematisches Diagramm einer exemplarischen Gesundheitskarte,
- Figur 12: ein schematisches Blockdiagramm einer exemplarischen Gesundheitskarte,
- Figur 13: ein schematisches Blockdiagramm eines exemplarischen Terminals, und
- Figur 14: ein schematisches Diagramm eines exemplarischen Systems.

Elemente der nachfolgenden Ausführungsformen, die einander entsprechen, werden mit denselben Bezugszeichen gekennzeichnet.

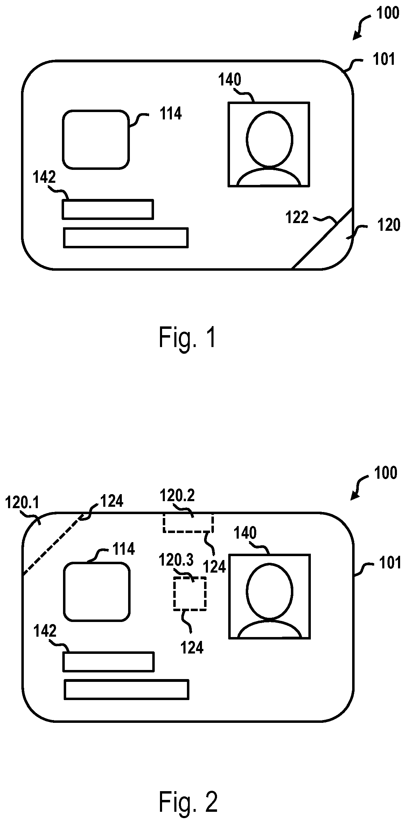

Figur 1 zeigt eine exemplarische elektronische Gesundheitskarte 100 mit einem Kartenkörper 101 und einem markierten Abschnitt 120 des Kartenkörpers 101, welcher durch eine Grenzmarkierung 122 begrenzt wird. Ist der markierte Abschnitt 120 vorhanden, zeigt dies eine Zustimmung des Inhabers der elektronischen Gesundheitskarte 100 zu einer Organspende an. Im Falle der Figur 1 ist der exemplarisch als ein Eckabschnitt des Kartenkörpers 101 gezeigt. markierte Abschnitt 120 Der markierte Abschnitt 120 kann beispielsweise visuell zusätzlich farblich hervorgehoben sein, etwa in Grün, und/oder beschriftet sein. Beispielsweise kann der markierte Abschnitt 120 mit einer erläuternden Beschriftung, etwa "Organspende ja" beschriftet sein. Durch ein Abtrennen des markierten Abschnitts 120 entlang der Grenzmarkierung 122 wird eine Ablehnung einer Organspende angezeigt. Das Abtrennen kann beispielsweise per Hand oder mit einem Hilfsmittel, wie etwa einer Scherre, erfolgen. Ferner umfasst die elektronische Gesundheitskarte 100 beispielsweise eine Portraitansicht 140 des Inhabers der elektronischen Gesundheitskarte 100 sowie eine Beschriftung 142. Die Beschriftung 142 umfasst beispielsweise Angaben zu dem Inhaber der elektronischen Gesundheitskarte 100 sowie zu seiner Krankenversicherung. Schließlich umfasst die elektronische Gesundheitskarte 100 beispielsweise noch eine kontaktbasierte Kommunikationsschnittstelle 116, über welche beispielsweise ein Terminal in der elektronischen Gesundheitskarte 100 gespeicherte Daten, etwa personenbezogene und/oder versicherungsbezogene Daten, auslesen kann.

Figur 2 zeigt die exemplarische elektronische Gesundheitskarte 100 aus Figur 1 mit alternativen Anordnungsmöglichkeiten für den markierten Abschnitt. Beispielsweise kann der markierte Abschnitt 120.1 als Eckabschnitt implementiert sein. Der markierte Abschnitt 120.1 wird dabei von eines Sollbruchlinie 124 begrenzt. Bei der Sollbruchlinie 124 kann es sich beispielsweise um eine Kerbe oder eine Perforation handeln, welche ein Abtrennen des markierten Abschnitt 120.1 von dem Kartenkörper per Hand ermöglicht. Ferner kann der markierte Abschnitt 120.2 beispielsweise am Rand des Kartenköpers implementiert sein, sodass ein Entfernen des markierten Abschnitt 120.2 in einer Aussparung im Rand des Kartenköpers 101 resultiert. Der markierte Abschnitt 120.2 wird ebenfalls von einer Sollbruchlinie begrenzt. Ein Entfernen eines markierten Abschnitts, wie etwa des markierten Abschnitts 120.1 oder 120.2, resultiert in einer Änderung des Umrisses des Kartenkörpers 101, welche beispielsweise von einem Terminal erfasst werden kann. Ferner kann der markierte Abschnitt 120.3 beispielsweise vollständig innerhalb des Kartenkörpers 101 angeordnet sein, sodass der markierten Abschnitts 120.3 vollständig von der Sollbruchlinie 124 umgrenzt wird und ein Entfernen des markierten Abschnitts 120.3 in einer Ausnehmung in dem Kartenköper 101 resultiert. Auch eine solche Ausnehmung kann von einem Terminal erfasst werden. Die Erfassung solcher Änderungen des Kartenkörpers 101 kann beispielsweise durch einen Sensor erfolgen, welcher bei einem Vorhandensein des markierten Abschnitts durch diesen betätigt wird. Alternativ kann ein Erfassen eines Vorhandenseins des markierten Abschnitts auch visuell mittels eines visuellen Sensors erfasst werden, welcher ein Bild der Gesundheitskarte 100 oder eines Teilbereichs der Gesundheitskarte 100, welcher den markierten Abschnitt umfassen sollte, aufnimmt und dieses mittels Bilderkennung auf ein Vorhandensein oder Fehlen des markierten Abschnitts untersucht.

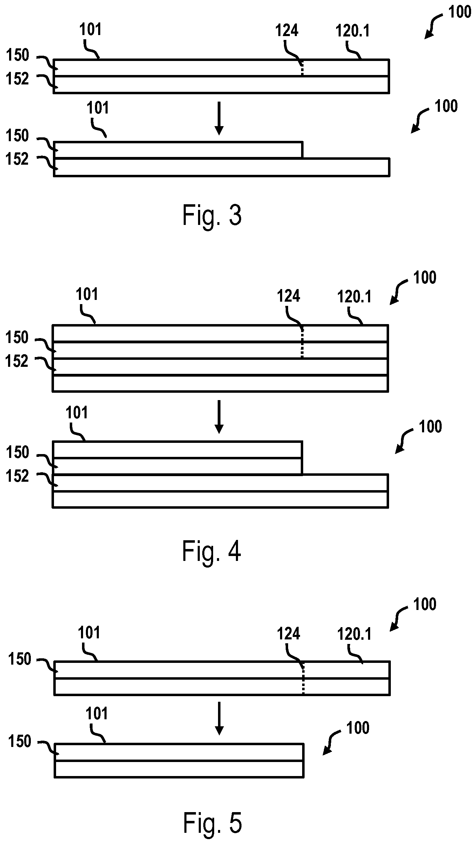

Figur 3 zeigt schematische Querschnitte durch eine exemplarisch Gesundheitskarte 100. Zunächst ist die Gesundheitskarte 100 mit markiertem Abschnitt 120.1 gezeigt. Die gezeigte Gesundheitskarte 100 umfasst einen Kartenkörper 101, bei welchem es sich um einen mehrschichtigen Verbundkörper handelt, welcher eine Mehrzahl von Materialschichten 150, 152 umfasst. Beispielsweise umfasst der Verbundkörper eine erste Materialschicht 150, durch welche sich der markierte Abschnitt 120.1 hindurcherstreckt, sowie eine zweite Materialschicht 152, durch welche sich der markierte Abschnitt 120.1 nicht hindurcherstreckt. Unter dem ersten Querschnitt der Gesundheitskarte 100 ist ein zweiter Querschnitt derselben Gesundheitskarte 100, nach einem Entfernen des markierten Abschnitt 120.1, gezeigt. Der Teil der ersten Materialschicht 150, durch welchen sich der markierte Abschnitt 120.1 hindurcherstreckt hat, fehlt nun. Die zweite Materialschicht 152, durch welche sich der markierte Abschnitt 120.1 nicht hindurcherstreckt hat, ist unverändert. Hierdurch ergibt sich, wie in Figur 3 zu sehen, beispielsweise eine Stufe im Kartenkörper 101. Beispielsweise kann sich auch eine Vertiefung und/oder ein Sackloch in dem Kartenkörper 101 ergeben, wenn der markierte Abschnitt von einem Rand des Kartenkörpers 101 entfernt angeordnet ist.

Figur 4 zeigt schematische Querschnitte durch eine weitere exemplarisch Gesundheitskarte 100. Zunächst ist die Gesundheitskarte 100 mit markiertem Abschnitt 120.1 gezeigt. Die gezeigte Gesundheitskarte 100 umfasst einen Kartenkörper 101, bei welchem es sich um einen mehrschichtigen Verbundkörper handelt, welcher eine Mehrzahl von Materialschichten 150, 152 umfasst. Beispielsweise umfasst der Verbundkörper mehrere erste Materialschichten 150, durch welche sich der markierte Abschnitt 120.1 hindurcherstreckt, sowie mehrere zweite Materialschicht 152, durch welche sich der markierte Abschnitt 120.1 nicht hindurcherstreckt. Unter dem ersten Querschnitt der Gesundheitskarte 100 ist ein zweiter Querschnitt derselben Gesundheitskarte 100, nach einem Entfernen des markierten Abschnitt 120.1, gezeigt. Die Teile der ersten Materialschichten 150, durch welche sich der markierte Abschnitt 120.1 hindurcherstreckt hat, fehlt nun. Die zweiten Materialschichten 152, durch welche sich der markierte Abschnitt 120.1 nicht hindurcherstreckt hat, sind unverändert. Hierdurch ergibt sich, wie in Figur 4 zu sehen, beispielsweise eine Stufe im Kartenkörper 101. Beispielsweise kann sich auch eine Vertiefung und/oder ein Sackloch in dem Kartenkörper 101 ergeben, wenn der markierte Abschnitt von einem Rand des Kartenkörpers 101 entfernt angeordnet ist.

Figur 5 zeigt schematische Querschnitte durch eine weitere exemplarisch Gesundheitskarte 100. Zunächst ist die Gesundheitskarte 100 mit markiertem Abschnitt 120.1 gezeigt. Die gezeigte Gesundheitskarte 100 umfasst einen Kartenkörper 101, bei welchem es sich um einen mehrschichtigen Verbundkörper handelt, welcher eine Mehrzahl von Materialschichten 150 umfasst. Beispielsweise erstreckt sich der markierte Abschnitt 120.1 durch alle Materialschichte des Kartenkörpers 101 hindurch. Unter dem ersten Querschnitt der Gesundheitskarte 100 ist ein zweiter Querschnitt derselben Gesundheitskarte 100, nach einem Entfernen des markierten Abschnitt 120.1, gezeigt. Es fehlt von allen Materialschichten 150 des Kartenkörpers 101 jeweils ein Teil, durch welchen sich der markierte Abschnitt 120.1 hindurcherstreckt hat. Es fehlt somit ein vollständiger Teil der Gesundheitskarte 100. Beispielsweise kann sich auch eine Durchgangsloch in dem Kartenkörper 101 ergeben, wenn der markierte Abschnitt von einem Rand des Kartenkörpers 101 entfernt angeordnet ist.

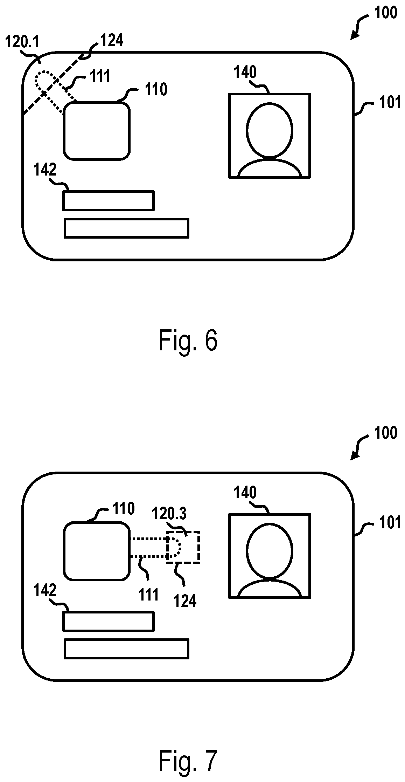

Figur 6 zeigt eine exemplarische elektronische Gesundheitskarte 100, etwa der Gesundheitskarte 100 aus Figur 1, mit einer Leiterschleife 111, welche sich durch den markierten Abschnitt 120.1 erstreckt. Beispielsweise kann der markierte Abschnitt 120.1 als Eckabschnitt implementiert sein. Der markierte Abschnitt 120.1 wird dabei von eines Sollbruchlinie 124 begrenzt. Bei der Sollbruchlinie 124 kann es sich beispielsweise um eine Kerbe oder eine Perforation handeln, welche ein Abtrennen des markierten Abschnitt 120.1 von dem Kartenkörper per Hand ermöglicht.

Ein Entfernen des markierten Abschnitts 120.1, resultiert in einer Änderung des Umrisses des Kartenkörpers 101, welche beispielsweise von einem Terminal erfasst werden kann. Die Erfassung einer solchen Änderung des Kartenkörpers 101 kann beispielsweise durch einen externen Sensor, etwa einen Sensor eines Terminals, erfolgen, welcher bei einem Vorhandensein des markierten Abschnitts 120.1 durch diesen betätigt wird. Alternativ kann ein Erfassen eines Vorhandenseins des markierten Abschnitts 120.1 auch visuell mittels eines externen visuellen Sensors, etwa einen visuellen Sensor eines Terminals, erfasst werden, welcher ein Bild der Gesundheitskarte 100 oder eines Teilbereichs der Gesundheitskarte 100, welcher den markierten Abschnitt 120.1 umfassen sollte, aufnimmt und dieses mittels Bilderkennung auf ein Vorhandensein oder Fehlen des markierten Abschnitts 120.1 untersucht.

Beispielsweise handelt es sich bei dem Kartenkörper 101 der Gesundheitskarte 100 um einen mehrschichtigen Verbundkörper, bei welchem sich die Leiterschleife 111 innerhalb einer der Materialschichten des mehrschichtigen Verbundkörpers erstreckt. Falls es sich bei der Sollbruchlinie 124 um eine Kerbe handelt, so erstreckt sich die Kerbe senkrecht zu einer Erstreckungsrichtung der Gesundheitskarte 100 bzw. des Kartenkörpers 101 der Gesundheitskarte 100. Beispielsweise erstreckt sich die Kerbe senkrecht nicht durch die Materialschicht mit der Leiterschleife 111 erstreckt, sondern nur durch ein oder mehrere oberhalb der die Materialschicht mit der Leiterschleife 111 angeordnete Materialschichten des Kartenkörpers 101.

Falls es sich bei der Sollbruchlinie 124 um eine Perforation handeln, ist die Perforation beispielsweise so angeordnet bzw. konfiguriert, dass sie die Leiterschleife 11 nicht unterbricht. Beispielsweise erstreckt sich die Leiterschleife durch Materialstege der zwischen dem markierte Abschnitt 120.1 und der restlichen Gesundheitskarte 100.

Die Leiterschleife 111 ist beispielsweise mit dem Prozessor 110 der Gesundheitskarte 100 elektrisch leitend verbunden. Der Prozessor 110 wird mit Hilfe der Leiterschleife in die Lage versetzt ein Vorhandensein oder Fehlen des markierten Abschnitts 120.1 zu erfassen. Ist die Leiterschleife 111 bzw. deren elektrische Leitfähigkeit unterbrochen, so fehlt der markierte Abschnitt 120.1. Ist die Leiterschleife 111 bzw. deren elektrische Leitfähigkeit nicht unterbrochen, so ist der markierte Abschnitt 120.1 vorhanden. Die elektrische Leitfähigkeit der Leiterschleife 111 kann der Prozessor 110 beispielsweise dadurch testen, dass er versucht einen Strom durch diese zu schicken. Ist der Prozessor 110 damit erfolgreich, ist die Leiterschleife 111 ununterbrochen. Ist der Prozessor 111 damit nicht erfolgreich, so ist die Leiterschleife 111 unterbrochen.

Beispielsweise sendet ein Terminal eine Anfrage an die Gesundheitskarte 100, beispielsweise über ein kontaktlose oder kontaktbasierte Kommunikationsschnittstelle der Gesundheitskarte 100. Auf den Empfang der Anfrage hin, testet die Gesundheitskarte 100 bzw. der Prozessor 110 der Gesundheitskarte 100 die elektrische Leitfähigkeit der Leiterschleife 111. Der Prozessor 110 erfasst ein Fehlen des markierten Abschnitts 120.1 beispielsweise anhand einer Unterbrechung der Leiterschleife 111 bzw. deren elektrischer Leitfähigkeit, während der Prozessor 110 ein Vorhandensein des markierten Abschnitts 120.1 beispielsweise daran erkennt, dass die Leiterschleife 111 bzw. deren elektrische Leitfähigkeit nicht unterbrochen ist. Das Ergebnis des Tests sendet die Gesundheitskarte 100 in Antwort auf die Anfrage des Terminals, beispielsweise über ihre kontaktlose oder kontaktbasierte Kommunikationsschnittstelle, an das Terminal. Beispielsweise ist die Antwort der Gesundheitskarte 100 mit einem kryptographischen Signaturschlüssel der Gesundheitskarte. Beispielsweis ist ein entsprechender Signaturschlüssel der Gesundheitskarte 100 in einem Speicher der Gesundheitskarte 100, insbesondere in einem geschützten Speicherbereich eines Speichers der Gesundheitskarte 100, gespeichert. Ferner erfolgt die Kommunikation zwischen der Gesundheitskarte 100 und dem Terminal beispielsweise über eine verschlüsselte Kommunikationsverbindung, insbesondere über eine mittels einer Ende-zu-Ende-Verschlüsselung verschlüsselten Kommunikationsverbindung. Beispielsweise ist die Kommunikationsverbindung symmetrisch und/oder asymmetrisch verschlüsselt.

Figur 7 zeigt eine weitere exemplarische elektronische Gesundheitskarte 100, etwa der Gesundheitskarte 100 aus Figur 1, mit einer alternativen Anordnung von einer Leiterschleife 111 und einem markierten Abschnitt. Die Leiterschleife 111 erstreckt sich durch den markierten Abschnitt 120.3. Der markierte Abschnitt 120.3 wird dabei von eines Sollbruchlinie 124 begrenzt. Bei der Sollbruchlinie 124 kann es sich beispielsweise um eine Kerbe oder eine Perforation handeln, welche ein Abtrennen des markierten Abschnitt 120.3 von dem Kartenkörper per Hand ermöglicht. Beispielsweise kann der markierte Abschnitt 120.3 vollständig innerhalb des Kartenkörpers 101 angeordnet sein, sodass der markierten Abschnitts 120.3 vollständig von der Sollbruchlinie 124 umgrenzt wird.

Ein Entfernen des markierten Abschnitts 120.3 resultiert in einer Ausnehmung in dem Kartenköper 101. Eine solche Ausnehmung in dem Kartenkörper 101 kann beispielsweise von einem Terminal erfasst werden. Die Erfassung einer solchen Änderung des Kartenkörpers 101 kann beispielsweise durch einen externen Sensor, etwa einen Sensor eines Terminals, erfolgen, welcher bei einem Vorhandensein des markierten Abschnitts 120.3 durch diesen betätigt wird. Alternativ kann ein Erfassen eines Vorhandenseins des markierten Abschnitts 120.3 auch visuell mittels eines externen visuellen Sensors, etwa einen visuellen Sensor eines Terminals, erfasst werden, welcher ein Bild der Gesundheitskarte 100 oder eines Teilbereichs der Gesundheitskarte 100, welcher den markierten Abschnitt 120.3 umfassen sollte, aufnimmt und dieses mittels Bilderkennung auf ein Vorhandensein oder Fehlen des markierten Abschnitts 120.3 untersucht.

Beispielsweise handelt es sich bei dem Kartenkörper 101 der Gesundheitskarte 100 um einen mehrschichtigen Verbundkörper, bei welchem sich die Leiterschleife 111 innerhalb einer der Materialschichten des mehrschichtigen Verbundkörpers erstreckt. Falls es sich bei der Sollbruchlinie 124 um eine Kerbe handelt, so erstreckt sich die Kerbe senkrecht zu einer Erstreckungsrichtung der Gesundheitskarte 100 bzw. des Kartenkörpers 101 der Gesundheitskarte 100. Beispielsweise erstreckt sich die Kerbe senkrecht nicht durch die Materialschicht mit der Leiterschleife 111 erstreckt, sondern nur durch ein oder mehrere oberhalb der die Materialschicht mit der Leiterschleife 111 angeordnete Materialschichten des Kartenkörpers 101.

Falls es sich bei der Sollbruchlinie 124 um eine Perforation handeln, ist die Perforation beispielsweise so angeordnet bzw. konfiguriert, dass sie die Leiterschleife 11 nicht unterbricht. Beispielsweise erstreckt sich die Leiterschleife durch Materialstege der zwischen dem markierte Abschnitt 120.3 und der restlichen Gesundheitskarte 100.

Die Leiterschleife 111 ist beispielsweise mit dem Prozessor 110 der Gesundheitskarte 100 elektrisch leitend verbunden. Der Prozessor 110 wird mit Hilfe der Leiterschleife in die Lage versetzt ein Vorhandensein oder Fehlen des markierten Abschnitts 120.3 zu erfassen. Ist die Leiterschleife 111 bzw. deren elektrische Leitfähigkeit unterbrochen, so fehlt der markierte Abschnitt 120.3. Ist die Leiterschleife 111 bzw. deren elektrische Leitfähigkeit nicht unterbrochen, so ist der markierte Abschnitt 120.3 vorhanden. Die elektrische Leitfähigkeit der Leiterschleife 111 kann der Prozessor 110 beispielsweise dadurch testen, dass er versucht einen Strom durch diese zu schicken. Ist der Prozessor 110 damit erfolgreich, ist die Leiterschleife 111 ununterbrochen. Ist der Prozessor 111 damit nicht erfolgreich, so ist die Leiterschleife 111 unterbrochen.

Beispielsweise sendet ein Terminal eine Anfrage an die Gesundheitskarte 100, beispielsweise über ein kontaktlose oder kontaktbasierte Kommunikationsschnittstelle der Gesundheitskarte 100. Auf den Empfang der Anfrage hin, testet die Gesundheitskarte 100 bzw. der Prozessor 110 der Gesundheitskarte 100 die elektrische Leitfähigkeit der Leiterschleife 111. Der Prozessor 110 erfasst ein Fehlen des markierten Abschnitts 120.3 beispielsweise anhand einer Unterbrechung der Leiterschleife 111 bzw. deren elektrischer Leitfähigkeit, während der Prozessor 110 ein Vorhandensein des markierten Abschnitts 120.3 beispielsweise daran erkennt, dass die Leiterschleife 111 bzw. deren elektrische Leitfähigkeit nicht unterbrochen ist. Das Ergebnis des Tests sendet die Gesundheitskarte 100 in Antwort auf die Anfrage des Terminals, beispielsweise über ihre kontaktlose oder kontaktbasierte Kommunikationsschnittstelle, an das Terminal. Beispielsweise ist die Antwort der Gesundheitskarte 100 mit einem kryptographischen Signaturschlüssel der Gesundheitskarte. Beispielsweis ist ein entsprechender Signaturschlüssel der Gesundheitskarte 100 in einem Speicher der Gesundheitskarte 100, insbesondere in einem geschützten Speicherbereich eines Speichers der Gesundheitskarte 100, gespeichert. Ferner erfolgt die Kommunikation zwischen der Gesundheitskarte 100 und dem Terminal beispielsweise über eine verschlüsselte Kommunikationsverbindung, insbesondere über eine mittels einer Ende-zu-Ende-Verschlüsselung verschlüsselten Kommunikationsverbindung. Beispielsweise ist die Kommunikationsverbindung symmetrisch und/oder asymmetrisch verschlüsselt.

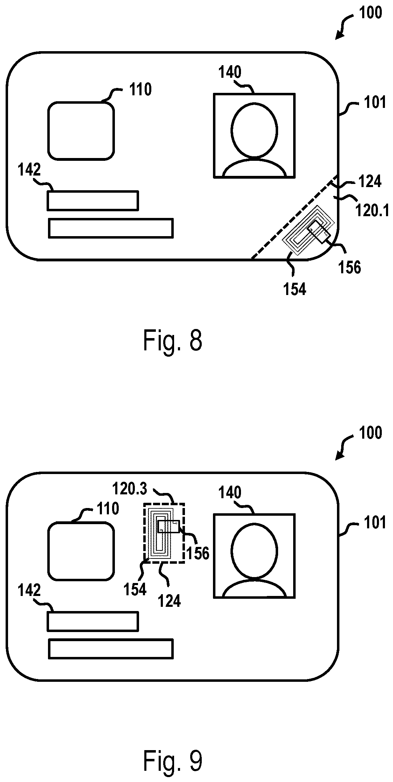

Figur 8 zeigt eine weitere exemplarische elektronische Gesundheitskarte 100, etwa der Gesundheitskarte 100 aus Figur 1. Die Gesundheitskarte 100 umfasst einen markierten Abschnitt 120.1 mit einer Schaltung. Der markierte Abschnitt 120.1 erstreckt sich zwischen einer Sollbruchlinie 124 und einem Rand des Kartenkörpers 101 der Gesundheitskarte 100. Die Schaltung umfasst eine Antenne 154 und ein mit der Antenne 154 elektrisch leitend verschaltetes Leuchtmittel 156. Die Schaltung ist dazu konfiguriert, das Leuchtmittel 156 mittels einer induktiven Energieeinkopplung elektromagnetischer Energie in die Antenne 154 zum Leuchten zu bringen. Eine Energieeinkopplung kann beispielsweise durch ein Terminal im Zuge einer Prüfung der Gesundheitskarte 100 erfolgen. Durch die induktive Energieeinkopplung elektromagnetischer Energie in die Antenne 154 kann das Leuchtmittel 156, beispielsweise eine Diode, von außen zum Leuchten gebracht werden kann. Durch Einkoppeln elektromagnetischer Energie in die Antenne 154 des Leuchtmittels kann mithin in einfacher Weise überprüft werden, ob der markierte Abschnitt 120.1 noch vorhanden ist. Ist der markierte Abschnitt 120.1 vorhanden, ist das leuchtende Leuchtmittel 156 vorhanden. Fehlt der markierte Abschnitt 120.1, fehlt das Leuchtmittel 156 und es kann kein Leuchten erfasst werden. Eine Energieeinkopplung kann beispielsweise durch ein Terminal im Zuge einer Prüfung der Gesundheitskarte erfolgen.

Figur 9 zeigt eine weitere exemplarische elektronische Gesundheitskarte 100, etwa der Gesundheitskarte 100 aus Figur 1. Die Gesundheitskarte 100 umfasst einen markierten Abschnitt 120.3 mit einer Schaltung, welcher vollständig von einer Sollbruchlinie 124 umgrenzt wird. Der markierte Abschnitt 120.3 erstreckt sich zwischen einer Sollbruchlinie 124 und einem Rand des Kartenkörpers 101 der Gesundheitskarte 100. Die Schaltung umfasst eine Antenne 154 und ein mit der Antenne 154 elektrisch leitend verschaltetes Leuchtmittel 156. Die Schaltung ist dazu konfiguriert, das Leuchtmittel 156 mittels einer induktiven Energieeinkopplung elektromagnetischer Energie in die Antenne 154 zum Leuchten zu bringen. Eine Energieeinkopplung kann beispielsweise durch ein Terminal im Zuge einer Prüfung der Gesundheitskarte 100 erfolgen. Durch die induktive Energieeinkopplung elektromagnetischer Energie in die Antenne 154 kann das Leuchtmittel 156, beispielsweise eine Diode, von außen zum Leuchten gebracht werden kann. Durch Einkoppeln elektromagnetischer Energie in die Antenne 154 des Leuchtmittels kann mithin in einfacher Weise überprüft werden, ob der markierte Abschnitt 120.3 noch vorhanden ist. Ist der markierte Abschnitt 120.3 vorhanden, ist das leuchtende Leuchtmittel 156 vorhanden. Fehlt der markierte Abschnitt 120.3, fehlt das Leuchtmittel 156 und es kann kein Leuchten erfasst werden. Eine Energieeinkopplung kann beispielsweise durch ein Terminal im Zuge einer Prüfung der Gesundheitskarte erfolgen.

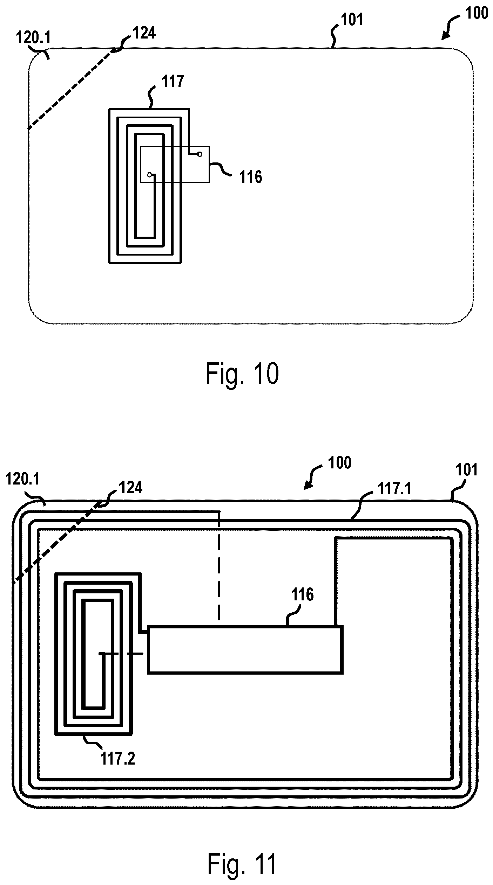

Figur 10 zeigt eine exemplarische elektronische Gesundheitskarte 100 mit einer kontaktlosen Kommunikationsschnittstelle 116, welche eine Antenne 117 umfasst. Die Antenne 117 kann beispielsweise zu einer kontaktlosen Kommunikation mit einem Terminal konfiguriert sein. Ferner kann die Antenne 117 beispielsweise zu einem Energy Harvesting via Induktion von Energie konfiguriert sein, welche das Terminal zu diesem Zweck sendet. Beispielsweise kann der markierte Abschnitt 120.1 beispielsweise so angeordnet sein, dass ein Entfernen des markierten Abschnitts 120.1 die Funktionsfähigkeit der Antenne 117 nicht beeinträchtigt. Beispielsweise kann der markierte Abschnitt 120.1 beispielsweise so angeordnet sein, dass die Antenne 117 sich nicht durch den markierten Abschnitt 120.1 erstreckt.

Figur 11 zeigt eine exemplarische elektronische Gesundheitskarte 100 mit einer kontaktlosen Kommunikationsschnittstelle 116, welche eine Mehrzahl von Antennen 117.1, 117.2 umfasst. Beispielweise umfasst die Gesundheitskarte 100 zwei Antennen 117.1, 117.2, von denen eine der beiden Antennen 117.1 durch den markierten Abschnitt 120.1 erstreckt, sodass ein Entfernen des markierten Abschnitts 120.1 die Antenne 120.1 unterbricht und deren Funktionsfähigkeit nachhaltig stört. Die zweite Antenne 120.2 erstreckt sich beispielsweise nicht durch den markierten Abschnitt 120.2, sodass deren Funktionsfähigkeit selbst bei einem Entfernen des markierten Abschnitts 120.1 vollständig erhalten bleibt. Somit wird beispielsweise die elektronische Gesundheitskarte 100 dazu in die Lage versetzt, anhand der Funktionsfähigkeit der Antenne 120.1 elektronisch zu erfassen, ob der markierte Abschnitt 120.1 vorhanden ist oder entfernt wurde. Beispielsweise kann die über die beiden Antennen 117.1, 117.2 induzierte Spannung im Zuge eines Energy Harvesting erfasst werden. Weicht der für die Antennen 117.1 erfasst Wert dabei deutlich von einem Sollwert ab, kann daraus auf ein Fehlen des markierten Abschnitts 120.1 geschlossen werden. Ebenso kann das Terminal beispielsweise die von der elektronischen Gesundheitskarte 100 im Zuge einer kontaktlosen Kommunikation übertragene Energie erfassen. Weicht diese infolge einer Funktionsunfähigkeit der Antenne 117.1 deutlich von einem zu erwartenden Wert ab, weil nur eine von zwei Antennen sendet, so kann daraus auf ein fehlen des markierten Abschnitts 120.1 geschlossen werden. Für eine Vergleichbarkeit der Messungen kann die elektronischen Gesundheitskarte 100 zu diesem Zwecke beispielsweise an einer vordefinierten Position relativ zu dem Terminal positioniert werden. Beispielsweise legt das Terminal die entsprechende Position fest, indem es für die elektronische Gesundheitskarte 100 eine Auflagefläche oder eine Aufnahme bereitstellt. Ferner kann die elektronische Gesundheitskarte 100 beispielsweise dazu konfiguriert sein, über die Antenne 117.2 eine Abfrage des Organspenderstatus des Inhabers der elektronischen Gesundheitskarte 100 zu empfangen und diese mit einem vordefinierten Signal, welches über die Antenne 117.1 gesendet wird zu beantworten. Empfängt das Terminal das entsprechende Signal, weiß es, dass die Antenne 117.1 funktioniert und der markierte Abschnitt 120.1 vorhanden ist. Empfängt das Terminal das entsprechende Signal nicht, weiß es, dass die Antenne 117.1 funktioniert und der markierte Abschnitt 120.1 vorhanden ist. Ferner kann die elektronische Gesundheitskarte 100 noch ein weiteres Signal über die Antenne 117.2 als Empfangsbestätigung der Abfrage senden, sodass das Terminal weiß, dass die kontaktlose Kommunikation grundsätzlich funktioniert und ein Ausbleiben des vordefinierten Signals der Antenne 117.1 tatsächlich auf einem Entfernen des markierten Abschnitts 120.1 beruht.

Schließlich kann beispielsweise auch nur die Antenne 117.1 als einzige Antenne vorgesehen sein. In diesem Fall kann an einem Ausfall der kontaktlosen Kommunikation ein Entfernen des markierten Abschnitts 120.1 erkannt werden. Dies gilt insbesondere, falls eine kontaktbasierte Kommunikation zugleich funktioniert, etwa wenn die elektronische Gesundheitskarte 100 in einer Aufnahme des Terminals angeordnet ist.

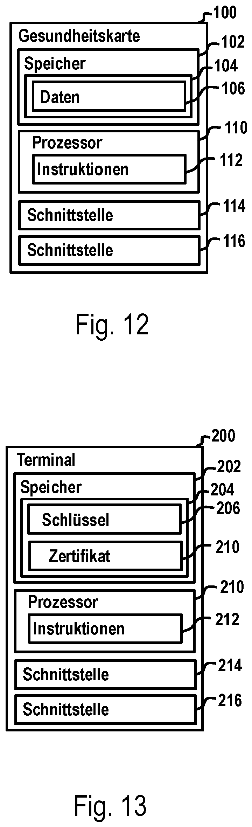

Figur 12 zeigt einen schematischen Aufbau einer elektronischen Gesundheitskarte 100. In einem Speicher 102 der elektronischen Gesundheitskarte 100, beispielsweise in einem geschützten Speicherbereich 104 sind Daten 106 gespeichert. Die Daten 106 umfassen beispielsweise personenbezogene oder versicherungsbezogene Daten des Inhabers der elektronischen Gesundheitskarte 100 umfassen. Diese Daten 106 kann ein Terminal beispielsweise Auslesen, nachdem es eine Leseberechtigung nachgewiesen hat. Hierzu verwendet das Terminal beispielsweise ein Berechtigungszertifikat, welches eine entsprechende Berechtigung belegt. Eine Übertragung der entsprechenden Daten 106 erfolgt beispielsweise über eine verschlüsselte Kommunikationsverbindung zwischen elektronischer Gesundheitskarte 100 und Terminal 200. Beispielsweise ist die Kommunikation mittels Ende-zu-Ende-Verschlüsselung verschlüsselt. Hierzu kann beispielsweise unter Verwendung asymmetrischer Schlüsselpaare der elektronischen Gesundheitskarte 100 und des Terminals 200 beispielsweise ein ephemererer symmetrischer Sitzungsschlüssel ausgehandelt werden, welcher zur Verschlüsselung der Kommunikation verwendet wird.

Ferner umfasst die elektronische Gesundheitskarte 100 beispielsweise einen Prozessor 110, welcher Programminstruktionen 112 ausführt. Ein Ausführen der entsprechenden Programminstruktionen 112 steuert beispielsweise eine Kommunikation zwischen elektronischer Gesundheitskarte 100 und Terminal. Insbesondere können die Programminstruktionen 112 kryptographische Protokolle bereitstellen und/oder zum Ansteuern von Antennen einer kontaktlosen Kommunikationsschnittstelle 116 der elektronischen Gesundheitskarte 100 dienen. Die Kommunikationsschnittstelle 116 kann ferner zum Energy Harvesting konfiguriert sein. Ferner kann die Kommunikationsschnittstelle 116 dazu konfiguriert sein, dass ihre Funktionsfähigkeit durch ein Entfernen des markierten Abschnitts des Kartenkörpers der elektronischen Gesundheitskarte 100 vollständig oder teilweise beeinträchtigt wird. Beispielsweise kann die Kommunikationsschnittstelle 116 dazu konfiguriert sein, dass ihre Funktionsfähigkeit durch ein Entfernen des markierten Abschnitts des Kartenkörpers der elektronischen Gesundheitskarte 100 nicht beeinträchtigt wird. Schließlich kann die elektronische Gesundheitskarte 100 eine kontaktbasierte Kommunikationsschnittstelle 114 umfassen. Beispielsweise kann die Kommunikationsschnittstelle 114 dazu konfiguriert sein, dass ihre Funktionsfähigkeit durch ein Entfernen des markierten Abschnitts des Kartenkörpers der elektronischen Gesundheitskarte 100 nicht beeinträchtigt wird.

Figur 13 zeigt einen schematischen Aufbau eines Terminals 200 zum Auslesen von Daten aus einer elektronischen Gesundheitskarte. Ferner kann das Terminal 200 zum Erfassen eines Vorhandenseins und/oder Fehlens eines markierten Abschnitts des Kartenkörpers der elektronischen Gesundheitskarte konfiguriert sein. Das Terminal 200 umfasst beispielsweise einen Speicher mit 202. In dem Speicher 202 sind beispielsweise in einem geschützten Speicherbereich kryptographische Schlüssel gespeichert. Diese kryptographischen Schlüssel umfassen beispielsweise eine privaten kryptographischen Schlüssel 206, welcher einem Berechtigungszertifikat 210 zugeordnet ist. Beispielsweise umfasst das entsprechende Berechtigungszertifikat 210 einen zu dem privaten kryptographischen Schlüssel 206 gehörenden öffentlichen kryptographischen Schlüssel und bestätigt für den Besitzer des privaten kryptographischen Schlüssels 206 eine Zugriffsberechtigung für einen Zugriff auf Daten, welche in einer elektronischen Gesundheitskarte gespeichert sind. Der Besitz des privaten kryptographischen Schlüssel 206, lässt sich beispielsweise durch eine Signatur mit dem privaten kryptographischen Schlüssel 206, etwa im Zuge eines Challenge-Response-Verfahrens nachweisen.

Ferner umfasst das Terminal 200 beispielsweise einen Prozessor 210, welcher Programminstruktionen 212 ausführt. Ein Ausführen der entsprechenden Programminstruktionen 212 steuert beispielsweise eine Kommunikation zwischen dem Terminal 200 und einer elektronischen Gesundheitskarte. Zur Kommunikation mit der elektronischen Gesundheitskarte umfasst das Terminal 200 beispielsweise eine kontaktbasierte Kommunikationsschnittstelle 214 und/oder eine kontaktlose Kommunikationsschnittstelle 216. Insbesondere können die Programminstruktionen 212 kryptographische Protokolle bereitstellen und/oder zum Ansteuern von ein oder mehreren Antennen der kontaktlosen Kommunikationsschnittstelle 216 des Terminals 200 und/oder zum Ansteuern der kontaktbasierten Kommunikationsschnittstelle 214 dienen. Die Kommunikationsschnittstelle 216 kann ferner zur Energieversorgung der elektronischen Gesundheitskarte mittels Energy Harvesting konfiguriert sein. Ferner kann die Kommunikationsschnittstelle 216 dazu konfiguriert sein, das Vorhandensein oder ein Fehlen eines markierten Abschnitts des Kartenkörpers der elektronischen Gesundheitskarte zu erfassen. Diese Erfassung basiert beispielsweise auf einer Erfassung einer Funktionsfähigkeit einer Antenne der elektronischen Gesundheitskarte, welche sich durch den markierten Abschnitt erstreckt. Schließlich kann die das Terminal 200 beispielsweise eine kontaktbasierte Kommunikationsschnittstelle 114 umfassen. Beispielsweise ist die kontaktbasierte Kommunikationsschnittstelle 114 in einer Aufnahme des Terminals 200 zum Aufnehmen der elektronischen Gesundheitskarte angeordnet. Beispielsweise sind die kontaktbasierte Kommunikationsschnittstelle 214 und/oder die kontaktlose Kommunikationsschnittstelle 216 dazu konfiguriert, das Vorhandensein oder ein Fehlen eines markierten Abschnitts des Kartenkörpers der elektronischen Gesundheitskarte mittels einer Abfrage der elektronischen Gesundheitskarte zu erfassen.

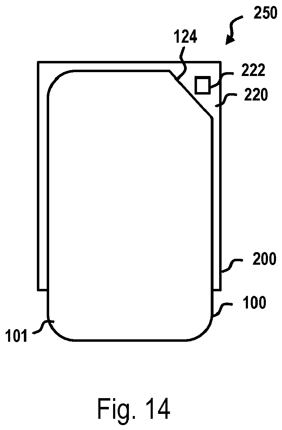

Figur 14 zeigt ein exemplarische System 250, welches ein Terminal 200 mit einer Aufnahme 220 zum Aufnehmen einer elektronischen Gesundheitskarte 100 sowie die elektronische Gesundheitskarte 100 umfasst. In der Aufnahme 220 ist ein Sensor 222 an der Stelle angeordnet, an der sich bei einer bestimmungsgemäßen Anordnung der elektronischen Gesundheitskarte 100 in der Aufnahme 220 der markierte Abschnitt 120 des Kartenkörpers 101 befindet. Ist der markierte Abschnitt 120 vorhanden wird der Sensor betätigt, fehlt der markierte Abschnitt 120 wie im Beispiel der Figur 14, so wird der Sensor nicht betätigt und dadurch das Fehlen des markierten Abschnitts 120 erfasst. Bei dem Sensor 222 kann es sich beispielsweise um einen mechanischen Sensor oder beispielsweise um einen elektromagnetischen Sensor, wie etwa eine Lichtschranke handeln. Beispielsweise handelt es sich bei dem Kartenkörper 101 der Gesundheitskarte 100 um einen mehrschichtigen Verbundkörper. Beispielsweise erstreckt sich der fehlende markierte Abschnitt 120 durch alle Materialschichten des mehrschichtigen Verbundkörpers hindurch, sodass infolge des Fehlens des markierten Abschnitts 120 ein vollständiges Stück des Kartenkörpers fehlt. Hierdurch kann sich beispielsweise der geometrische Umriss des Kartenkörpers ändern. Beispielsweise kann sich aus dem Fehlen des markierten Abschnitts 120 ein Durchgangsloch in dem Kartenkörper 101 ergeben. Beispielsweise erstreckt sich der markierte Abschnitt 120 durch ein oder mehrere Materialschichten des mehrschichtigen Verbundkörpers hindurch, während sich der markierte Abschnitt 120 durch ein oder mehrere weitere Materialschichten des Verbundkörpers nicht hindurch erstreckt. Beispielsweise kann sich aus dem Fehlen des markierten Abschnitts 120 an einem Rand des Verbundkörpers eine Stufe ergeben, ohne dass sich der geometrische Umriss der Gesundheitskarte 100 infolge des Fehlens des markierten Abschnitts 120 ändert. Beispielsweise kann sich aus dem Fehlen des markierten Abschnitts 120, welcher beabstandet von dem Rand des Verbundkörpers angeordnet ist, eine Vertiefung und/oder ein Sackloch innerhalb des Umrisses des Verbundkörpers ergeben.

Die Erfindung kann ohne Einschränkung und nur beispielhaft durch die folgenden Merkmalskombinationen weiter beschrieben werden. Die folgenden Merkmalskombinationen können bevorzugte Ausführungsformen umfassen.
1. Elektronische Gesundheitskarte, wobei die Gesundheitskarte einen Kartenkörper mit einem Prozessor, einem Speicher mit Programminstruktionen und einer Kommunikationsschnittstelle umfasst, wobei in dem Speicher ferner personenbezogene und krankenversicherungsbezogene Daten eines Inhabers der elektronischen Gesundheitskarte gespeichert sind, wobei der Prozessor dazu konfiguriert ist, die gespeicherten Daten unter Verwendung der Programminstruktionen zum Auslesen über die Kommunikationsschnittstelle bereitzustellen,
   wobei der Kartenkörper ferner in einem Ausgabezustand einen markierten Abschnitt umfasst,
   wobei das Vorhandensein des markierten Abschnitts eine Zustimmung des Inhabers zu einer Organspende anzeigt, wobei ein Entfernen des markierten Abschnitts eine Ablehnung des Inhabers zu der Organspende anzeigt.
2. Elektronische Gesundheitskarte nach Merkmalskombination 1, wobei zumindest ein Teil einer den markierten Abschnitt begrenzenden Grenzlinie als Sollbruchlinie ausgestaltet ist.
3. Elektronische Gesundheitskarte nach Merkmalskombination 2, wobei der Kartenkörper entlang der Sollbruchlinie eine im Verhältnis zu einem angrenzenden Bereich des Kartenkörpers reduzierte Materialstärke aufweist.
4. Elektronische Gesundheitskarte nach einer der Merkmalskombinationen 2 bis 3, wobei der Kartenkörper entlang der Sollbruchlinie eine Perforation aufweist.
5. Elektronische Gesundheitskarte nach einer der Merkmalskombinationen 2 bis 4, wobei die Sollbruchlinie zu einem manuellen Abrechen des markierten Abschnitts entlang der Sollbruchlinie mittels eines Sprödbruchs konfiguriert ist.
6. Elektronische Gesundheitskarte nach einer der Merkmalskombinationen 2 bis 4, wobei die Sollbruchlinie zu einem manuellen Abrechen des markierten Abschnitts entlang der Sollbruchlinie mittels eines Verformungsbruchs konfiguriert ist.
7. Elektronische Gesundheitskarte nach einer der vorangehenden Merkmalskombinationen, wobei es sich bei den Kartenkörper um einen mehrschichtigen Verbundkörper handelt, welcher eine Mehrzahl von Materialschichten umfasst,
   wobei sich der markierte Abschnitt durch ein oder mehrerer aufeinander angeordnete Materialschichten der Mehrzahl von Materialschichten hindurcherstreckt, während sich der markierte Abschnitt durch ein oder mehrere weitere Materialschichten der Mehrzahl von Materialschichten nicht hindurcherstreckt, oder
   wobei sich der markierte Abschnitt durch alle Materialschichten der Mehrzahl von Materialschichten hindurcherstreckt.
8. Elektronische Gesundheitskarte nach einer der Merkmalskombinationen 2 bis 7, wobei der markierte Abschnitt begrenzt ist von der Sollbruchlinie und einem Rand des Kartenkörpers.
9. Elektronische Gesundheitskarte nach Merkmalskombination 8, wobei durch ein Entfernen des markierten Abschnitts ein geometrischer Umriss des Kartenkörpers verändert wird.
10. Elektronische Gesundheitskarte nach Merkmalskombination 9, wobei es sich bei dem markierten Abschnitt um einen Eckabschnitt des Kartenkörpers handelt.
11. Elektronische Gesundheitskarte nach Merkmalskombination 9, wobei aus einem Entfernen des markierten Abschnitts eine Aussparung in einem Rand des Kartenkörpers resultiert.
12. Elektronische Gesundheitskarte nach einer der Merkmalskombinationen 2 bis 7, wobei der markierte Abschnitt vollständig von der Sollbruchlinie umgrenzt ist.
13. Elektronische Gesundheitskarte nach Merkmalskombination 12, wobei aus einem Entfernen des markierten Abschnitts eine Ausnehmung in dem Kartenkörper resultiert.
14. Elektronische Gesundheitskarte nach einem der voranstehenden Ansprüche, wobei die Gesundheitskarte eine Leiterschleife zum Erfassen, ob der markierte Abschnitt vorhanden ist, umfasst, wobei sich die Leiterschleife durch den markierten Abschnitt erstreckt, sodass die Leiterschleife bei einem Entfernen des markierten Abschnitts unterbrochen wird.
15. Elektronische Gesundheitskarte nach Merkmalskombination 14, wobei die Leiterschleife elektrisch leitend mit dem Prozessor verbunden ist, wobei der Prozessor konfiguriert ist zu prüfen, ob die Leiterschleife unterbrochen ist.
16. Elektronische Gesundheitskarte nach einem der voranstehenden Ansprüche, wobei der markierte Abschnitt eine Schaltung umfasst, wobei die Schaltung eine Antenne und ein mit der Antenne elektrisch leitend verschaltetes Leuchtmittel umfasst, wobei die Schaltung dazu konfiguriert ist, das Leuchtmittel mittels einer induktiven Energieeinkopplung elektromagnetischer Energie in die Antenne zum Leuchten zu bringen.
17. Elektronische Gesundheitskarte nach einem der voranstehenden Ansprüche, wobei die Kommunikationsschnittstelle eine oder mehrere Antennen umfasst und zu einer kontaktlosen Kommunikation mit einem Terminal konfiguriert ist.
18. Elektronische Gesundheitskarte nach Merkmalskombination 17, wobei die Antennenführung der ein oder mehreren Antennen und eine Positionierung des markierten Abschnitts so aufeinander abgestimmt sind, dass sich keine der Antennen durch den markierten Abschnitt erstreckt.
19. Elektronische Gesundheitskarte nach Merkmalskombination 17, wobei die Kommunikationsschnittstelle genau eine Antenne umfasst, welche sich durch den markierten Abschnitt erstreckt.
20. Elektronische Gesundheitskarte nach Merkmalskombination 19, wobei die Kommunikationsschnittstelle ferner zu einer kontaktbasierten Kommunikation mit einem Terminal konfiguriert ist, wobei durch ein Fehlschlagen der kontaktlosen Kommunikation mit dem Terminal infolge eines Fehlens des markierten Abschnitts, während die kontaktbasierte Kommunikation erfolgreich ist, dem Terminal gegenüber eine Ablehnung des Inhabers zu der Organspende signalisiert wird.
21. Elektronische Gesundheitskarte nach Merkmalskombination 17, wobei die Kommunikationsschnittstelle zumindest zwei Antenne umfasst, von denen sich eine erste Antenne durch den markierten Abschnitt erstreckt, während sich eine zweite Antenne nicht durch den markierten Abschnitt erstreckt.
22. Elektronische Gesundheitskarte nach Merkmalskombination 21, wobei die beiden Antennen für eine induktive Energieeinkopplung elektromagnetischer Energie des Terminals in die Gesundheitskarte konfiguriert sind, wobei der Prozessor dazu konfiguriert ist einen relativen Abfall der eingekoppelten Energie der ersten Antenne gegenüber der zweiten Antenne zu erfassen,
   wobei der Prozessor ferner dazu konfiguriert ist, anhand eines Überschreitens eines ersten vordefinierten Schwellenwerts durch den erfassten relativen Abfall ein Fehlen des markierten Abschnitts zu detektieren,
   wobei der Prozessor auf ein Detektieren des Fehlens des markierten Abschnitts hin ein Ablehnungssignal erzeugt und an das Terminal sendet, wobei das Ablehnungssignal eine Ablehnung des Inhabers zu der Organspende anzeigt.
23. Elektronische Gesundheitskarte nach Merkmalskombination 21, wobei die elektronische Gesundheitskarte ferner dazu konfiguriert ist unter Verwendung der beiden Antennen eine vordefinierte Energiemenge abstrahlen, wobei ein Abfall der abgestrahlten Energie infolge einer Unterbrechung der ersten Antenne bei einer Anordnung der elektronischen Gesundheitskarte an einer vordefinierten Position relative zu dem Terminal, welcher in einem Abfall des von dem Terminal empfangbaren Teils der abgestrahlten Energie unter einen zweiten vordefinierten Schwellenwert resultiert, gegenüber dem Terminal ein Fehlen des markierten Abschnitts anzeigt.
24. Elektronische Gesundheitskarte nach Merkmalskombination 21, wobei der Prozessor der elektronischen Gesundheitskarte ferner zu Folgendem konfiguriert ist:
   Empfangen einer Abfrage eines Organspenderstatus des Inhabers der elektronischen Gesundheitskarte von dem Terminal über die zweite Antenne,
   in Antwort auf die empfangene Abfrage, Erzeugen eines ersten vordefinierten Signals,
   Initiieren eines Sendens des vordefinierten Signals an das Terminal über die erste Antenne, wobei das erste vordefinierte Signal eine Zustimmung des Inhabers zu einer Organspende signalisiert.
25. Elektronische Gesundheitskarte nach Merkmalskombination 24, wobei der Prozessor der elektronischen Gesundheitskarte ferner zu Folgendem konfiguriert ist:
   in Antwort auf die empfangene Abfrage, Erzeugen eines zweiten vordefinierten Signals,
   Senden des zweiten vordefinierten Signals an das Terminal über die zweite Antenne, wobei das zweite vordefinierte Signal eine Bestätigung des Empfangens der Abfrage durch die elektronische Gesundheitskarte signalisiert.
26. Elektronische Gesundheitskarte nach einer der Merkmalskombinationen 24 bis 25, wobei eine Voraussetzung für das Antworten auf die Abfrage des Organspenderstatus durch die elektronische Gesundheitskarte ein erfolgreicher Nachweis einer Berechtigung zum Abfragen des Organspenderstatus durch das Terminal gegenüber der elektronischen Gesundheitskarte ist.
27. Elektronische Gesundheitskarte nach einer der vorangehenden Merkmalskombinationen, wobei eine Voraussetzung für das Bereitstellen der Daten zum Auslesen durch das Terminal ein erfolgreicher Nachweis einer Berechtigung zum Auslesen der Daten durch das Terminal gegenüber der elektronischen Gesundheitskarte ist.
28. Terminal, wobei das Terminal einen Prozessor, einen Speicher mit Programminstruktionen und eine Kommunikationsschnittstelle umfasst, wobei der Prozessor dazu konfiguriert ist, in einer Gesundheitskarte gespeicherte personenbezogene und krankenversicherungsbezogene Daten eines Inhabers der elektronischen Gesundheitskarte unter Verwendung der Programminstruktionen über die Kommunikationsschnittstelle auszulesen,
   wobei das Terminal ferner konfiguriert ist zum Erfassen, ob ein Abschnitt des Kartenkörpers der elektronischen Gesundheitskarte vorhanden ist, dessen Vorhandensein eine Zustimmung des Inhabers zu einer Organspende anzeigt, wobei ein Fehlen des Abschnitts eine Ablehnung des Inhabers zu der Organspende anzeigt.
29. Terminal nach Merkmalskombination 28, wobei das Terminal eine Aufnahme umfasst, welche zum Aufnehmen der elektronischen Gesundheitskarte konfiguriert ist, wobei in der Aufnahme ein Sensor angeordnet ist, welcher dazu konfiguriert ist, das Vorhandensein des Abschnitts des Kartenkörpers der elektronischen Gesundheitskarte zu erfassen.
30. Terminal nach Merkmalskombination 28, wobei die Kommunikationsschnittstelle des Terminals zu einer kontaktlosen Kommunikation mit der elektronischen Gesundheitskarte konfiguriert ist.
31. Terminal nach Merkmalskombination 30, wobei die Kommunikationsschnittstelle des Terminals ferner zu einer kontaktbasierten Kommunikation mit der elektronischen Gesundheitskarte konfiguriert ist, wobei der Prozessor des Terminals ferner zu Folgendem konfiguriert ist:
   zum Ausführen eines ersten Versuchs eines kontaktlosen Aufnehmens einer Kommunikation mit der elektronischen Gesundheitskarte und eines zweiten Versuchs eines kontaktbasierten Aufnehmens einer Kommunikation mit der elektronischen Gesundheitskarte,
   falls der erste Versuch fehlschlägt, während der zweite Versuch erfolgreich ist, Erzeugen eines Ablehnungsvermerks, dass eine Ablehnung des Inhabers zu der Organspende vorliegt.
32. Terminal nach Merkmalskombination 30, wobei das Terminals ferner konfiguriert ist zu einem induktiven Energieeinkoppeln elektromagnetischer Energie in die elektronische Gesundheitskarte, wobei der Prozessor des Terminals ferner sowohl zu einem Empfangen eines Ablehnungssignal, welches eine Ablehnung des Inhabers der elektronischen Gesundheitskarte zu der Organspende anzeigt, als auch zu einem Empfangen eines Zustimmungssignals konfiguriert ist, welches eine Zustimmung des Inhabers der elektronischen Gesundheitskarte zu der Organspende anzeigt.
33. Terminal nach Merkmalskombination 30, wobei der Prozessor des Terminals ferner zu Folgendem konfiguriert ist:
   Erzeugen einer Abfrage des Organspenderstatus eines Inhabers der elektronischen Gesundheitskarte,
   Senden der Abfrage an die elektronische Gesundheitskarte unter Verwendung der Kommunikationsschnittstelle des Terminals, wobei ein Empfangen eines ersten vordefinierten Signals von der elektronischen Gesundheitskarte in Antwort auf das Senden der Abfrage ein Vorhandensein des markierten Abschnitts anzeigt.
34. Terminal nach Merkmalskombination 33, wobei ein Empfangen eines zweiten vordefinierten Signals von der elektronischen Gesundheitskarte in Antwort auf das Senden der Abfrage den gesundheitskartenseitigen Empfang der Abfrage bestätigt.
35. Terminal nach Merkmalskombination 30, wobei der Prozessor des Terminals ferner zu Folgendem konfiguriert ist:
   unter Verwendung der Kommunikationsschnittstelle des Terminals Bestimmen einer Signalstärke, mit welcher Signale der elektronischen Gesundheitskarte im Zuge einer kontaktlosen Kommunikation von dem Terminal empfangen werden, wobei die elektronische Gesundheitskarte an einer vordefinierten Position relativ zu dem Terminal angeordnet ist, und,
   falls die Signalstärke einen vordefinierten Schwellenwert unterschreitet, Erzeugen eines Ablehnungsvermerks, dass eine Ablehnung des Inhabers zu der Organspende vorliegt.
36. Terminal nach Merkmalskombination 28, wobei der Prozessor des Terminals ferner zu Folgendem konfiguriert ist:
   Erzeugen einer Abfrage des Organspenderstatus eines Inhabers der elektronischen Gesundheitskarte,
   Senden der Abfrage an die elektronische Gesundheitskarte unter Verwendung der Kommunikationsschnittstelle des Terminals,
   Empfangen einer Antwort der elektronischen Gesundheitskarte, welche das Ergebnis einer Prüfung der elektronischen Gesundheitskarte umfasst, ob eine sich durch den markierten Abschnitt des Kartenkörpers der Gesundheitskarte erstreckende Leiterschleife unterbrochen ist, wobei das Ergebnis der Prüfung anzeigt, ob der Abschnitt der elektronischen Gesundheitskarte vorhanden ist.
37. System umfassend eine elektronische Gesundheitskarte nach einer der Merkmalskombinationen 1 bis 27 sowie ein Terminal nach einer der Merkmalskombinationen 28 bis 36.

### Bezugszeichenliste

- 100: Gesundheitskarte
- 101: Kartenkörper
- 102: Speicher
- 104: geschützter Speicherbereich
- 106: Daten
- 110: Prozessor
- 111: Leiterschleife
- 112: Programminstruktionen
- 114: kontaktbasierte Kommunikationsschnittstelle
- 116: kontaktlose Kommunikationsschnittstelle
- 117: Antenne
- 120: markierter Abschnitt
- 122: Grenzmarkierung
- 124: Sollbruchlinie
- 140: Portraitbild
- 142: Beschriftung
- 150: Materialschicht
- 152: Materialschicht
- 154: Antenne
- 156: Leuchtmittel
- 200: Terminal
- 202: Speicher
- 204: geschützter Speicherbereich
- 206: kryptographischer Schlüssel
- 208: Zertifikat
- 210: Prozessor
- 212: Programminstruktionen
- 214: kontaktbasierte Kommunikationsschnittstelle
- 216: kontaktlose Kommunikationsschnittstelle
- 220: Aufnahme
- 222: Sensor
- 250: System

## Patentansprüche

1. Elektronische Gesundheitskarte (100), wobei die Gesundheitskarte (100) einen Kartenkörper (101) mit einem Prozessor (110), einem Speicher (102) mit Programminstruktionen (112) und einer Kommunikationsschnittstelle (114, 116) umfasst, wobei in dem Speicher (102) ferner personenbezogene und krankenversicherungsbezogene Daten (106) eines Inhabers der elektronischen Gesundheitskarte (100) gespeichert sind, wobei der Prozessor (110) dazu konfiguriert ist, die gespeicherten Daten unter Verwendung der Programminstruktionen (112) zum Auslesen über die Kommunikationsschnittstelle (114, 116) bereitzustellen,
wobei der Kartenkörper (101) ferner in einem Ausgabezustand einen markierten Abschnitt (120) umfasst,
wobei das Vorhandensein des markierten Abschnitts (120) eine Zustimmung des Inhabers zu einer Organspende anzeigt, wobei ein Entfernen des markierten Abschnitts (120) eine Ablehnung des Inhabers zu der Organspende anzeigt.

2. Elektronische Gesundheitskarte (100) nach Anspruch 1, wobei zumindest ein Teil einer den markierten Abschnitt (120) begrenzenden Grenzlinie als Sollbruchlinie (124) ausgestaltet ist.

3. Elektronische Gesundheitskarte (100) nach Anspruch 2, wobei der Kartenkörper (101) entlang der Sollbruchlinie (124) eine im Verhältnis zu einem angrenzenden Bereich des Kartenkörpers (101) reduzierte Materialstärke aufweist und/oder
wobei der Kartenkörper (101) entlang der Sollbruchlinie (124) eine Perforation aufweist.

4. Elektronische Gesundheitskarte (100) nach einem der Ansprüche 2 bis 3, wobei die Sollbruchlinie (124) zu einem manuellen Abrechen des markierten Abschnitts (120) entlang der Sollbruchlinie (124) mittels eines Sprödbruchs konfiguriert ist oder
wobei die Sollbruchlinie (124) zu einem manuellen Abrechen des markierten Abschnitts (120) entlang der Sollbruchlinie (124) mittels eines Verformungsbruchs konfiguriert ist.

5. Elektronische Gesundheitskarte (100) nach einem der vorangehenden Ansprüche, wobei es sich bei den Kartenkörper (101) um einen mehrschichtigen Verbundkörper handelt, welcher eine Mehrzahl von Materialschichten (150, 152) umfasst,
wobei sich der markierte Abschnitt (120) durch ein oder mehrerer aufeinander angeordnete Materialschichten (150) der Mehrzahl von Materialschichten (150, 152) hindurcherstreckt, während sich der markierte Abschnitt (120) durch ein oder mehrere weitere Materialschichten (152) der Mehrzahl von Materialschichten (150, 152) nicht hindurcherstreckt, oder
wobei sich der markierte Abschnitt (120) durch alle Materialschichten (150, 152) der Mehrzahl von Materialschichten (150, 152) hindurcherstreckt.

6. Elektronische Gesundheitskarte (100) nach einem der Ansprüche 2 bis 5, wobei der markierte Abschnitt (120) begrenzt ist von der Sollbruchlinie (124) und einem Rand des Kartenkörpers (101).

7. Elektronische Gesundheitskarte (100) nach Anspruch 6, wobei durch ein Entfernen des markierten Abschnitts (120) ein geometrischer Umriss des Kartenkörpers (101) verändert wird,
wobei es sich bei dem markierten Abschnitt (120.1) beispielsweise um einen Eckabschnitt des Kartenkörpers (101) handelt und/oder
wobei aus einem Entfernen des markierten Abschnitts (120.2) beispielsweise eine Aussparung in einem Rand des Kartenkörpers (101) resultiert.

8. Elektronische Gesundheitskarte (100) nach einem der Ansprüche 2 bis 5, wobei der markierte Abschnitt (120) vollständig von der Sollbruchlinie (124) umgrenzt ist,
wobei aus einem Entfernen des markierten Abschnitts (120.3) beispielsweise eine Ausnehmung in dem Kartenkörper (101) resultiert.

9. Elektronische Gesundheitskarte (100) nach einem der voranstehenden Ansprüche, wobei die Gesundheitskarte (100) eine Leiterschleife (111) zum Erfassen, ob der markierte Abschnitt (120) vorhanden ist, umfasst, wobei sich die Leiterschleife (111) durch den markierten Abschnitt (120) erstreckt, sodass die Leiterschleife bei einem Entfernen des markierten Abschnitts (120) unterbrochen wird,
wobei die Leiterschleife (111) beispielsweise elektrisch leitend mit dem Prozessor (110) verbunden ist, wobei der Prozessor (110) beispielsweise konfiguriert ist zu prüfen, ob die Leiterschleife (111) unterbrochen ist, und/oder
wobei der markierte Abschnitt (120) eine Schaltung umfasst, wobei die Schaltung eine Antenne (154) und ein mit der Antenne (154) elektrisch leitend verschaltetes Leuchtmittel (156) umfasst, wobei die Schaltung dazu konfiguriert ist, das Leuchtmittel (156) mittels einer induktiven Energieeinkopplung elektromagnetischer Energie in die Antenne (154) zum Leuchten zu bringen.

10. Elektronische Gesundheitskarte (100) nach einem der voranstehenden Ansprüche, wobei die Kommunikationsschnittstelle (116) eine oder mehrere Antennen (117) umfasst und zu einer kontaktlosen Kommunikation mit einem Terminal (200) konfiguriert ist.

11. Elektronische Gesundheitskarte (100) nach Anspruch 10, wobei die Antennenführung der ein oder mehreren Antennen (117) und eine Positionierung des markierten Abschnitts (120) so aufeinander abgestimmt sind, dass sich keine der Antennen (117) durch den markierten Abschnitt (120) erstreckt und/oder
wobei die Kommunikationsschnittstelle (116) genau eine Antenne (117.1) umfasst, welche sich durch den markierten Abschnitt (120) erstreckt,
wobei die Kommunikationsschnittstelle (114) beispielswiese ferner zu einer kontaktbasierten Kommunikation mit einem Terminal (200) konfiguriert ist, wobei durch ein Fehlschlagen der kontaktlosen Kommunikation mit dem Terminal (200) infolge eines Fehlens des markierten Abschnitts (120), während die kontaktbasierte Kommunikation erfolgreich ist, dem Terminal (200) gegenüber beispielsweise eine Ablehnung des Inhabers zu der Organspende signalisiert wird,
wobei die Kommunikationsschnittstelle (116) zumindest zwei Antenne (117.1, 117.2) umfasst, von denen sich eine erste Antenne (117.1) durch den markierten Abschnitt (120) erstreckt, während sich eine zweite Antenne (117.2) nicht durch den markierten Abschnitt (120) erstreckt,
wobei die beiden Antennen (117.1, 117.2) beispielsweise für eine induktive Energieeinkopplung elektromagnetischer Energie des Terminals (200) in die Gesundheitskarte (100) konfiguriert sind, wobei der Prozessor (110) beispielsweise dazu konfiguriert ist einen relativen Abfall der eingekoppelten Energie der ersten Antenne (117.1) gegenüber der zweiten Antenne (117.2) zu erfassen,
wobei der Prozessor (110) beispielsweise ferner dazu konfiguriert ist, anhand eines Überschreitens eines ersten vordefinierten Schwellenwerts durch den erfassten relativen Abfall ein Fehlen des markierten Abschnitts (120) zu detektieren,
wobei der Prozessor (110) auf ein Detektieren des Fehlens des markierten Abschnitts (120) hin beispielsweise ein Ablehnungssignal erzeugt und an das Terminal (200) sendet, wobei das Ablehnungssignal eine Ablehnung des Inhabers zu der Organspende anzeigt, oder
wobei die elektronische Gesundheitskarte (100) beispielsweise ferner dazu konfiguriert ist unter Verwendung der beiden Antennen (117.1, 117.2) eine vordefinierte Energiemenge abstrahlen, wobei ein Abfall der abgestrahlten Energie infolge einer Unterbrechung der ersten Antenne (117.1) bei einer Anordnung der elektronischen Gesundheitskarte (100) an einer vordefinierten Position relative zu dem Terminal (200), welcher in einem Abfall des von dem Terminal (200) empfangbaren Teils der abgestrahlten Energie unter einen zweiten vordefinierten Schwellenwert resultiert, gegenüber dem Terminal (200) beispielsweise ein Fehlen des markierten Abschnitts (120) anzeigt, oder
wobei der Prozessor (110) der elektronischen Gesundheitskarte (100) beispielsweise ferner zu Folgendem konfiguriert ist:
Empfangen einer Abfrage eines Organspenderstatus des Inhabers der elektronischen Gesundheitskarte (100) von dem Terminal (200) über die zweite Antenne (117.2),
in Antwort auf die empfangene Abfrage, Erzeugen eines ersten vordefinierten Signals,
Initiieren eines Sendens des vordefinierten Signals an das Terminal (200) über die erste Antenne (117.1), wobei das erste vordefinierte Signal eine Zustimmung des Inhabers zu einer Organspende signalisiert.

12. Terminal (200), wobei das Terminal (200) einen Prozessor (210), einen Speicher (202) mit Programminstruktionen (212) und eine Kommunikationsschnittstelle (214, 216) umfasst, wobei der Prozessor (210) dazu konfiguriert ist, in einer Gesundheitskarte (100) gespeicherte personenbezogene und krankenversicherungsbezogene Daten (106) eines Inhabers der elektronischen Gesundheitskarte (100) unter Verwendung der Programminstruktionen (212) über die Kommunikationsschnittstelle (214, 216) auszulesen,
wobei das Terminal (200) ferner konfiguriert ist zum Erfassen, ob ein Abschnitt (120) des Kartenkörpers (101) der elektronischen Gesundheitskarte (100) vorhanden ist, dessen Vorhandensein eine Zustimmung des Inhabers zu einer Organspende anzeigt, wobei ein Fehlen des Abschnitts (120) eine Ablehnung des Inhabers zu der Organspende anzeigt.

13. Terminal (200) nach Anspruch 26, wobei das Terminal (200) eine Aufnahme (220) umfasst, welche zum Aufnehmen der elektronischen Gesundheitskarte (100) konfiguriert ist, wobei in der Aufnahme (220) ein Sensor (222) angeordnet ist, welcher dazu konfiguriert ist, das Vorhandensein des Abschnitts (120) des Kartenkörpers (101) der elektronischen Gesundheitskarte (100) zu erfassen, oder
wobei die Kommunikationsschnittstelle (216) des Terminals (200) zu einer kontaktlosen Kommunikation mit der elektronischen Gesundheitskarte (100) konfiguriert ist,
wobei die Kommunikationsschnittstelle (214) des Terminals (200) beispielsweise ferner zu einer kontaktbasierten Kommunikation mit der elektronischen Gesundheitskarte (100) konfiguriert ist, wobei der Prozessor (210) des Terminals (200) beispielsweise ferner zu Folgendem konfiguriert ist:
zum Ausführen eines ersten Versuchs eines kontaktlosen Aufnehmens einer Kommunikation mit der elektronischen Gesundheitskarte (100) und eines zweiten Versuchs eines kontaktbasierten Aufnehmens einer Kommunikation mit der elektronischen Gesundheitskarte (100),
falls der erste Versuch fehlschlägt, während der zweite Versuch erfolgreich ist, Erzeugen eines Ablehnungsvermerks, dass eine Ablehnung des Inhabers zu der Organspende vorliegt, oder
wobei das Terminals (200) beispielsweise ferner konfiguriert ist zu einem induktiven Energieeinkoppeln elektromagnetischer Energie in die elektronische Gesundheitskarte (100), wobei der Prozessor (210) des Terminals (200) beispielsweise ferner sowohl zu einem Empfangen eines Ablehnungssignal, welches eine Ablehnung des Inhabers der elektronischen Gesundheitskarte (100) zu der Organspende anzeigt, als auch zu einem Empfangen eines Zustimmungssignals konfiguriert ist, welches eine Zustimmung des Inhabers der elektronischen Gesundheitskarte (100) zu der Organspende anzeigt, oder
wobei der Prozessor (210) des Terminals (200) beispielsweise ferner zu Folgendem konfiguriert ist:
Erzeugen einer Abfrage des Organspenderstatus eines Inhabers der elektronischen Gesundheitskarte (100),
Senden der Abfrage an die elektronische Gesundheitskarte (100) unter Verwendung der Kommunikationsschnittstelle (216) des Terminals (200), wobei ein Empfangen eines ersten vordefinierten Signals von der elektronischen Gesundheitskarte (100) in Antwort auf das Senden der Abfrage ein Vorhandensein des markierten Abschnitts (120) anzeigt.
wobei der Prozessor (210) des Terminals (200) beispielsweise ferner zu Folgendem konfiguriert ist:
unter Verwendung der Kommunikationsschnittstelle (216) des Terminals (200) Bestimmen einer Signalstärke, mit welcher Signale der elektronischen Gesundheitskarte (100) im Zuge einer kontaktlosen Kommunikation von dem Terminal (200) empfangen werden, wobei die elektronische Gesundheitskarte (100) an einer vordefinierten Position relativ zu dem Terminal (200) angeordnet ist, und,
falls die Signalstärke einen vordefinierten Schwellenwert unterschreitet, Erzeugen eines Ablehnungsvermerks, dass eine Ablehnung des Inhabers zu der Organspende vorliegt.

14. Terminal (200) nach Anspruch 12, wobei der Prozessor (210) des Terminals (200) ferner zu Folgendem konfiguriert ist:
Erzeugen einer Abfrage des Organspenderstatus eines Inhabers der elektronischen Gesundheitskarte (100),
Senden der Abfrage an die elektronische Gesundheitskarte (100) unter Verwendung der Kommunikationsschnittstelle (214, 216) des Terminals (200),
Empfangen einer Antwort der elektronischen Gesundheitskarte (100), welche das Ergebnis einer Prüfung der elektronischen Gesundheitskarte (100) umfasst, ob eine sich durch den markierten Abschnitt (120) des Kartenkörpers (101) der Gesundheitskarte (100) erstreckende Leiterschleife (111) unterbrochen ist, wobei das Ergebnis der Prüfung anzeigt, ob der Abschnitt (120) der elektronischen Gesundheitskarte (100) vorhanden ist.

15. System (250) umfassend eine elektronische Gesundheitskarte (100) nach einem der Ansprüche 1 bis 11 sowie ein Terminal (200) nach einem der Ansprüche 12 bis 14.
